# EUROPEAN PATENT APPLICATION

(11) **EP 3 546 582 A1**
(43) Date of publication of application: **02.10.2019**
(21) Application number: 18164080.6
(22) Date of filing: 26.03.2018
(51) Int. Cl.: C12N 15/82

(54) **PROMOTER ACTIVATING ELEMENTS**

(71) Applicant: KWS SAAT SE & Co. KGaA, 37574 Einbeck (DE)
(72) Inventor: Streitner, Corinna, 49328 Melle (DE); Weltmeier, Fridtjof, 37574 Einbeck (DE)

(57) **Abstract**

The present invention relates to promoter activating nucleic acid sequences, which are able to increase expression of a nucleic acid molecule of interest in a cell or an organism upon site-specific introduction into a recipient promoter controlling the expression of the nucleic acid molecule of interest. The invention also provides methods to identify such promoter activating elements and methods to introduce them into an organism or a cell to specifically increase the expression of a nucleic acid molecule of interest. Furthermore, the invention also relates to the use of the promoter activating elements to increase the expression of a nucleic acid molecule of interest.

SEQUENCE LISTING

<110> KWS SAAT SE

<120> Promoter activating elements

<130> KWS0289EP

<160> 40

<170> BiSSAP 1.3.6

<210> 1
<211> 57
<212> DNA
<213> Zea mays

<400> 1
aacccggacc cggtaggaag gagctataaa gacaagccaa acgagggcat cccttct        57

<210> 2
<211> 57
<212> DNA
<213> Zea mays

<400> 2
cgctataaaa tatccccacg ctgcttcgcc ctgcccacca cagcatccgc agttccc        57

<210> 3
<211> 57
<212> DNA
<213> Zea mays

<400> 3
tgctgttagc ggtataaaaa gcggaaaccc tagcattcgc cgcgagctta tcactta        57

<210> 4
<211> 60
<212> DNA
<213> Zea mays

<400> 4
gggactcggc gacaggcctt ttgtagaccg cagccggcac catctcttgc cgcacccccc      60

<210> 5
<211> 60
<212> DNA
<213> Zea mays

<400> 5
cccctcttaa aagccgcctc tcgccgccgc ccgcaaaccc tcatttttct ctctcctgcg      60

<210> 6

<211> 60
<212> DNA
<213> Zea mays

<400> 6
ccaccataaa tgcgccgcgg ccgtcctcgc tgcccaaccc ttgctcgctg cgccgccgcc     60

<210> 7
<211> 60
<212> DNA
<213> Zea mays

<400> 7
ggcgttaata tctcccctcc cttccctctt ctggtctccg ccccgctcct tgcctccgat     60

<210> 8
<211> 60
<212> DNA
<213> Zea mays

<400> 8
cgtttttttt acgctgtcaa tgcataacct gcgttggcat tccgcctgct ggacttcctc     60

<210> 9
<211> 60
<212> DNA
<213> Zea mays

<400> 9
cgcccgccgt cataaatagc cagccccatc cccagcttct ttccccaacc tcatcttctc     60

<210> 10
<211> 60
<212> DNA
<213> Zea mays

<400> 10
cgcccgccgt aataaataga caccccctcc acaccctctt tccccaacct cgtgttgttc     60

<210> 11
<211> 60
<212> DNA
<213> Zea mays

<400> 11
tgctgctagc agtataaata tgctgaaagc ctgaaaccct aggcgaagct tatcgcttat     60

<210> 12
<211> 60
<212> DNA
<213> Zea mays

<400> 12
gtcggcttta aaaggacacg agcgcttaaa cccccacccc atatccgcat ccgctgcctc     60

<210> 13
<211> 60
<212> DNA
<213> Zea mays

<400> 13
gccggcttta aaaacgcaca caagcgctaa aaccctctcc accgtccacc tcagctccca     60

<210> 14
<211> 60
<212> DNA
<213> Zea mays

<400> 14
cccgactaca tcaaccaacg cgtatcggcg gtggcaaacc ctctagcttc ccactccgct     60

<210> 15
<211> 20
<212> DNA
<213> Zea mays

<400> 15
tataaagaca agccaaacga                                                 20

<210> 16
<211> 20
<212> DNA
<213> Zea mays

<400> 16
gctataaaat atccccacgc                                                 20

<210> 17
<211> 20
<212> DNA
<213> Zea mays

<400> 17
gtataaaaag cggaaaccct                                                 20

<210> 18
<211> 20
<212> DNA
<213> Zea mays

<400> 18

gctataaaga caagccaaac                                                    20

<210> 19
<211> 20
<212> DNA
<213> Artificial Sequence

<220>
<223> fusion of two contiguous streches of E53

<400> 19
gctataaaga gcatcccttc                                                    20

<210> 20
<211> 60
<212> DNA
<213> Beta vulgaris

<400> 20
atgtaaaaaa aaagcttata taaagggaat cagacatgag gttttggcat aaaaactatc      60

<210> 21
<211> 60
<212> DNA
<213> Beta vulgaris

<400> 21
ccccctcacc cctacatata caccactctc tccttcaatc ttcttcatca ctctcatttt      60

<210> 22
<211> 60
<212> DNA
<213> Beta vulgaris

<400> 22
gcgtaattat gaacgttata taaaccggtt acaattacaa cctatcacac caaaaagcaa      60

<210> 23
<211> 60
<212> DNA
<213> Beta vulgaris

<400> 23
ccacctcctt caaacctatt tatactccct cacctccttc actacctcct cgcttcaccc      60

<210> 24
<211> 60
<212> DNA
<213> Beta vulgaris

<400> 24

tctacacttc ctttagtata tttagcctca aattactact ggtcacttat acatttctca     60

<210> 25
<211> 60
<212> DNA
<213> Beta vulgaris

<400> 25
gtcggtcaaa caagtcttta aatacagcct attcccttca ttggtttctc atccttcatt     60

<210> 26
<211> 60
<212> DNA
<213> Beta vulgaris

<400> 26
accctaaaac actccttata taattcactc cctcacattt caatttccgc ctcctatact     60

<210> 27
<211> 60
<212> DNA
<213> Beta vulgaris

<400> 27
gtttctctct ctctccttta aataaaaccc taactttctt caccactctc actcacactc     60

<210> 28
<211> 60
<212> DNA
<213> Beta vulgaris

<400> 28
cttcctctcc tcaacataat aaaggatagc aagtcacaca ttcaatcgcc tctctctcct     60

<210> 29
<211> 60
<212> DNA
<213> Beta vulgaris

<400> 29
gatactccat ttccattatt taaggagtgc aagtgtgggt gtatgaaagt aaggtaccaa     60

<210> 30
<211> 60
<212> DNA
<213> Beta vulgaris

<400> 30
ttcctcattt ctccagtata agaaccacca ccacccttgt tctcccacaa acgcaaaatc     60

<210> 31
<211> 1754
<212> DNA
<213> Zea mays

<400> 31
atcggaccca aatcatagac acatgatgat ataataacag acaaccaaaa ttgagagtgg 60
caaaatagca aatttctgat agtcatgtga tagagaatag tagacaattt tgacataata 120
tatgtacact aattagtcaa caaaagcgat attgcggtta aaacagtgat tgccagtgtt 180
ttgacccgag tgtcctaacc aaccaataaa gtaaatttat gctatgtgtc ctcgtccaga 240
tggatgatgc aagaagacac aagatttatt ttggttcgga caatagaagg cctactttca 300
gcggaggggg atgggattta tattatcttg cacctaagtg cttgtagtag aaggtacaag 360
ttagtcgaga gagagagaga atcccaactc tctgcggatg attgaggcaa gtgtcaatat 420
cggccgcgga gggcaatagg tgaagtgtat tgtcctcctc ccttgcaagc cttggactcc 480
ttttatagcc ttaatgaggg aatcaaggag taataattag ttgaagactg attaagaaac 540
agtccatctg ttagtttttt tgtttaaata ggctaaagct aattttatct agttcttaat 600
tagctaataa ttattatttc gtaggatcca aaccattcct aagctatagt gctattatat 660
caagtgtaga tctatatgta ctcaaggtca tgatgtttgc aaaccaacaa tgaaatttat 720
cgcacacatt ggtcatggca gatcaacttt tttgccacaa aacaaacaag aatagtgcaa 780
acgaagttgc ataaaatgaa acaatatatt atgtgaatag ttgcatggtt tatcttgcta 840
gttccatttt aacacacaca catatcttgc tagttccatt ttaacttcta cttgcacaat 900
tccaaaagga acctaaattt catttaccga tgagtcacaa gaaacttaga tctaattaaa 960
tttaaagaaa aatagcaata tttatatttt taaatatatt tattataaaa atttatctca 1020
tattctagct aatgatattt attatgcatc ataactatta aatatatagc tatatatata 1080
tatatttcat aagtttcatg ttgtttaact taatagagat ttatattttt agggctagtt 1140
tggcaaacta tttttccaaa ggattttcat ttctataaag aaaattattt ttttaaaaaa 1200
aatagaaatc tcttgaaaga atagaattgt taaactactc ttagacaaat aaagagtatc 1260
cttggttcgt ggctaaccgt atcatatttt atctaagtta gttgttccaa ttaaagaact 1320
aattttatac acaaaagtta agtaaagtat agcaaattag tccgcgaacc aaatatgacc 1380
gaaatatcga ggagtgagga ggcttaaccc ttcccatgtg tgtatctact gttacaccgt 1440
gagctacaaa gttactggca caaacgtata gaggatggtg aggacatggg aagataaaat 1500
cctggtccag caagatccgt tcttccaaat gggatcaggt gattggctcc agttcctcct 1560
cccctcagca ccaccagtct cctccagtcc agctcccgtc ttctccgcct caagagtctc 1620
agaccaacgg caaagttcta gaagcacggt tgcacgggca gcacggcata acacctccct 1680

ccactgatcc agttccagtc gcccaacgcc ccaacgtctt ctcttgcaaa tcgcaagcaa 1740

acttcctgtt cacg 1754

<210> 32
<211> 658
<212> DNA
<213> Zea mays

<400> 32
gttggctact tgagttagat tttggttgtg tttcatcccc acgtacgtcc agcaaagaaa 60

aattgaagct agtgcatgca tggttcgtca tcaaatgcat ggccggccgg atacaaattt 120

gaactgtagc tatcgacgta cgcatgtatt aatttatatc agagaagaca aggaacacag 180

atacatacat gtcgaaacaa tcattttcta tggcacttga gctagctagc atacaatttt 240

gttttaaatg aaatgaaact gaagacgatc gatcgaattg aaggttgtgg ttcgtgagca 300

atgcaatgca gtttcacaga acgttgccaa tgcaacaagc caccaagaaa agagaagtct 360

actcgatctt gcaatgatta ggcttggatg atgcgtgggg ccacgtacgt atggacatcg 420

aagaacccca tcctcagcgt gtggcctgag ggtgatggca aagctgatcc acacattgcg 480

gccccctttc ccccctcaga gaccctgacc tcccgagcac agccagccac cgcgcaacgc 540

cggccaccac caccaccacc atacctgcta gcgctagctc tctttattta acgccgccgt 600

gtgcgtgcct cgacgacctc actactttga gctgcaaggt ccgaactaaa aagcaccg 658

<210> 33
<211> 1700
<212> DNA
<213> Beta vulgaris

<400> 33
tataagttca aacttcaata caggtatttt cgggatgtga ttaccttaca atttctcatt 60

ttcaaagaat tttacctgtg cagctatgtt ggataacctg tgcgagattc cgtttcagta 120

ggacactttt tttttttacc aataaaaaaa aacttataag ttcatgagct aatttttata 180

gatagtttaa agtaccgggt ggaggatgaa tagttgagtt ttttcttcaa aattagatac 240

ttcctccgtt ttttattaga tgttacactt ttcaaatcac ggactcctag gtaatttttg 300

gagaggagag agatagagag aatgaaaaac aaaagggtcc catgtgagta tgtgatagga 360

gagagataga gagaatttat tacccaaaat aaaagtgtaa catctaattc aaaacttcct 420

aaaatagaaa gtgtaacatc taaaaaaaac ggaggaagta tttgaatttg atatagatat 480

tgtgtctttg tgtgtgttga atttcaattc ccagttccct aaaaaaaatt tacaattgca 540

atttcgagat tatgatgtaa attaaatttg agagactaga aagtatttgg tcaacccaaa 600

aaaaaaatat caatacttat ataaatcaaa aacataatag agaatccaat tttactaaaa 660

atattagtaa ttttgattaa aataatctat taaaatgaac tctaaccttc acataatttc 720

cacatattat taatcaacaa aataagcatc acaaattatt agaataggcg atctaatttt 780

aacataaaat tagacgaatt caaattgaat ttttctaaca agctcattcc atttcacgca 840

acccaaaatt atcctagtca gtagtcatcc attcttttct cattccttta ttcttgatta 900

tcgaactaca acagataatt tcaaaaaaaa actaaattgg tagtcttaac tgattaaact 960

acttactaaa tggattaaag aatgtcatta ctgaatagat taaactgatt acgaaataga 1020

ttaacttggt ccctaaatag attaaattag ttactatatt aaaattaggc gatctcttac 1080

aaaaccaact gaataagcat agctctgtat attacctaga tttcaactaa atcaaaaccc 1140

cttacagttc aatctagagc tgatcatttt ggctcggccc gtcccatttt tgggccgggt 1200

tttagtcaga tttttttggc ccgcggtcgg gcccggcccg atttttttgg ctttgggcaa 1260

gccaaaaacg acttttcagt ttattttttg gcccgacccg tttttacccg caaaagcccg 1320

ctaatttagg tccgcacttt gggcacaaaa atttagcccg aacttaaacc tggcccgacc 1380

catgatcacc tctagtttaa tccaaactaa aaaactacac aagttagcca aaaattatgt 1440

ctactttgta caactttata aaatacacac agtagttgat atcttgatga ttaactcctt 1500

ttgaagtttg actacacacc aaccccaaac acacccactt tttcccccct cttgtcacca 1560

accccccctc ctctttagcc accaaagttt ggttggtgag tcctccataa ctgctaaatt 1620

ctctcttttt tctctctcct aaaaaactaa aacccaccaa aatttcagac atcaaaaaaa 1680

ttacaagtga aggaaacaat 1700

<210> 34
<211> 991
<212> DNA
<213> Beta vulgaris

<400> 34
aaagaaggaa aggaaggaat ttgaacatgt gacctatcgt tcacagcacc tcaatcttaa 60

tcactagacc aaaacatcct tggttcttgc gcaagaaggt tggctagaaa ttttttgtaa 120

aaacactagc cccgctcagt tcataatgag aatgtcgatg tcaccaaagg gatattaaat 180

gaatggaatt gggatatgga tggaatataa tgaaatagag ccactttgag gttccctatg 240

aaatgaggca tggaagggag ccactacgaa aaagttccgg gagttacgaa ggaagcttcg 300

agctcatatt ggtcatgaac ccgattactg agtctaataa gttcaattga aaagaaaaag 360

tcttatgttc taaaagaact tttcgtgcgg tttgcatgag ttcatagtcc atataatata 420

atgcaggaat gaagttctca gttgattctt ccacacccgt ccctcacccc ctaggcccca 480

ccttcacccc gccgaaaaaa ataaagaaaa tccaacgtta tttttcttag aaatgacagt 540

ttgatataga aaggaaaaat aataataaaa aaaaaaagtg ttggcgtttt cattttcaac 600

ctcagtatgt tggtttgccc caacaagttc tgaaccaatt ggcgatgtaa tcttataaga 660

agaatctaac gttggtccat tttgcttcta cagttttgaa agttaggtgg gccccattat 720

tatgttgatc ctagaataat taattttggt aggctgagaa gaggaaaaat aaagaacaat 780

gctaaaaaca agtgaaaaat atagttgcaa ctcatgatgc aacatgagat gcgatgaaat 840

atgatagtaa cttgagctca caactctgta tataagtgct catttggaca cttattttct 900

acaatttcct agtaactcag cttagcttca ttcccgactt ttttataaaa gtcaggacga 960

tcaatatcta tctatttatc tgtctgtctg t 991

<210> 35
<211> 35
<212> DNA
<213> Zea mays

<220>
<221> misc_feature
<222> 10..32
<223> /note="n is a, c, g, or t"

<400> 35
ttattattan nnnnnnnnnn nnnnnnnnnn nntga 35

<210> 36
<211> 35
<212> DNA
<213> Artificial Sequence

<220>
<223> Zm-prom1v3

<220>
<221> misc_feature
<222> 10..32
<223> /note="n is a, c, g, or t"

<400> 36
gtataaaagn nnnnnnnnnn nnnnnnnnnn nntga 35

<210> 37
<211> 38
<212> DNA
<213> Zea mays

<400> 37
aagataaaat cctggtccag caagatccgt tcttccaa 38

<210> 38
<211> 40
<212> DNA

<213> Artificial Sequence

<220>
<223> ZmZEP1v1

<400> 38
aagtataaaa gtcctggtcc agcaagatcc gttcttccaa                              40

<210> 39
<211> 39
<212> DNA
<213> Artificial Sequence

<220>
<223> ZmZEP1v2

<400> 39
aagctataaa gagcatccct tcaagatccg ttcttccaa                               39

<210> 40
<211> 39
<212> DNA
<213> Artificial Sequence

<220>
<223> ZmZEP1v3

<400> 40
aagctataaa atatccccac gcaagatccg ttcttccaa                               39

## Description

### Technical Field

The present invention relates to novel promoter activating elements. These short nucleic acid sequences are able to increase expression of a nucleic acid molecule of interest in a cell or an organism upon site-specific introduction into a recipient promoter controlling the expression of the nucleic acid molecule of interest. Using this new technology allows to increase the expression of endogenous or exogenous nucleic acid molecules up to many fold of what is achieved under the control of a promoter without the introduced activating elements. The invention also provides methods to identify such promoter activating elements and methods to introduce them into an organism or a cell to specifically increase the expression of a nucleic acid molecule of interest. Furthermore, the invention also relates to the use of the promoter activating elements to increase the expression of a nucleic acid molecule of interest.

### Background of the invention

The expression levels of many genes in an organism depend on different factors such as developmental stages or physiologic and environmental conditions. The expression of one gene can be induced under certain circumstances and completely shut down if the circumstances change. The starting point for gene expression, the transcription of a gene, is regulated by a range of different mechanisms, which usually involve the promoter region harbouring the transcription start site (TSS). While some promoters are active in all circumstances (constitutive promoters), others are tightly regulated and only respond to certain stimuli. Transcription factors bind to specific DNA sequences and activate or repress transcription (trans-acting factors). Promoter sequences therefore carry a number of binding sites for trans-acting factors, so called cis-regulatory elements, but the functions of some sequence stretches found in promoters are not completely understood yet.

Being able to modulate the expression of certain genes in an organism opens up a range of opportunities to improve biotechnological processes or agricultural yields. Therefore, new technologies are continuously sought, which allow to specifically control expression levels of a target gene. Promoters are an obvious target for such approaches, but up to date, there is still little known about the possibilities of activating endogenous promoters by minimal modification. No generic approach for activation of gene expression by addition of for example ≤ 20bp elements is currently available.

It is known that increased expression can be achieved by using strong promoters, e.g. the 35S promoter. Different translation enhancing elements have been described to be useful for expressing high levels of protein in plant cells, as parts of transgenes, or in viral expression vectors (e.g. the 5' untranslated leader of tobacco mosaic virus RNA which consists of a 68-base sequence (see Ofoghi et al., 2005. Comparison of tobacco etch virus and tobacco mosaic virus enhancers for expression of human calcitonin gene in transgenic potato plant. In Key Engineering Materials (Vol. 277, pp. 7-11). Trans Tech Publications.). However, these elements are relatively large. The same is true for enhancing promoter elements like the 35S enhancer, or introns reported to increase expression, e.g. adh1 intron from Zea mays (Callis et al, 1987. Introns increase gene expression in cultured maize cells. Genes & development, 1(10), 1183-1200.).

Crop traits can be improved by increased ectopic expression of a trait gene. As an example, Sun et al. (Nature comm., 2017, doi: 10.1038/ncomms14752) reported that increased expression of maize PLASTOCHRON1 enhances biomass and seed yield. They increased expression by a transgenic approach, using the GA2ox promoter. This transgenic approach to increase ectopic expression of a trait gene has the limitation that planting of transgenic plants has high regulatory requirements.

Recently, Zhang et al. found in the genus Malus an allelic variation of the Iron-Regulated Transporter1 (IRT1) promoter in which a TATA box insertion has been identified (Plant Physiology, 2017, Vol. 173, 715-727, doi: 10.1104/pp.16.01504). Further results suggest that this insertion seems to be causative for a slight upregulation of the promoter activity (~1.5 fold). It is also possible that the increased promoter activity is caused by the increased expression of the potential TATA-box binding proteins TFIID, which activates also the IRT1 promoter. However, the insertion element has not been isolated and it has not been investigated whether it can be introduced into a different promoter and still shows enhancer activity. Furthermore, the level of upregulation is rather low and thus not sufficient for many applications where a significant increase of expression levels is required to obtain a phenotypic or metabolic effect of interest.

It was an aim of the present invention to provide a widely applicable technology to enhance the expression of any specific gene of interest in a cell or an organism in significant manner.

It was thus an aim of the present invention that the expression of the gene of interest should be increased by at least 2-fold of what is achieved under the control of strong promoters known in the prior art, preferably at least 5-fold, at least 10-fold, at least 20-fold, at least 30-fold, or at least 40-fold.

Furthermore, it was an aim of the present invention that the provided technology should only require a minimal modification of the involved sequences, preferably 20 nucleotides or less than 20 nucleotides should be added, deleted or substituted.

### Summary of the invention

The present invention relates to several aspects to establish a new technology to increase the expression of endogenous or exogenous nucleic acid molecules up to many times by introducing promoter activating sequences into the promoter.

The above identified objectives have been achieved by providing, in a first aspect, a promoter activating nucleic acid sequence configured for targeted site-specific insertion into a recipient promoter controlling the expression of a nucleic acid molecule of interest in a cell or an organism, wherein the promoter activating nucleic acid sequence causes an increased expression of the nucleic acid molecule of interest upon site-specific insertion, preferably wherein the nucleic acid molecule of interest is heterologous or native to the recipient promoter and/or is an endogenous or exogenous nucleic acid molecule to the cell or organism.

In one embodiment, the promoter activating nucleic acid sequence has a length between 6 and 70 nucleotides, preferably between 7 and 60 nucleotides, more preferably between 8 and 40 nucleotides and most preferably between 9 and 20 nucleotides.

In another embodiment, the promoter activating nucleic acid sequence described in any of the embodiments above comprises or consist of one or more contiguous stretch(es) of nucleotides isolated from a donor promoter, wherein the donor promoter is a promoter of a gene having a high expression level.

In a further embodiment, each of the one or more contiguous stretch(es) described above is at least 90%, 91%, 92%, 93%, 94%, 95%, 96%, 97%, 98%, or 99% identical to or is identical to the core promoter sequence of the donor promoter over the whole length of the one or more contiguous stretch(es).

In yet another embodiment, each of the one or more contiguous stretch(es) described in any of the embodiments above is at least 90%, 91%, 92%, 93%, 94%, 95%, 96%, 97%, 98%, or 99% identical to or is identical to a sequence of the same length from position -50 to position +20 relative to the transcription start site of the donor promoter over the whole length of each stretch.

In another embodiment, each of the one or more contiguous stretch(es) described in any of the embodiments above has a length of six or more nucleotides.

In a further embodiment, the promoter activating nucleic acid sequence described in any of the embodiments above comprises one or more TATA box motif(s) of the donor promoter or one or more TATA box motif(s) having a relative score of greater than 0.8, greater than 0.81, greater than 0.81, greater than 0.82, greater than 0.83, greater than 0.84, greater than 0.85, greater than 0.86, greater than 0.87, greater than 0.88, greater than 0.89, greater than 0.90, greater than 0.91, greater than 0.92, greater than 0.93, greater than 0.94, greater than 0.95, greater than 0.96, greater than 0.97, greater than 0.98, or greater than 0.99 when matching or aligning the one or more TATA box motif(s) to TATA box consensus.

In one embodiment, the promoter activating nucleic acid sequence described in any of the embodiments above comprises one or more pyrimidine patch (Y patch) promoter element(s) of the donor promoter.In yet another embodiment, the promoter activating nucleic acid sequence described in any of the embodiments above comprises one or more TATA box motif(s) of the donor promoter or one or more TATA box motif(s) having a relative score of greater than 0.8, greater than 0.81, greater than 0.81, greater than 0.82, greater than 0.83, greater than 0.84, greater than 0.85, greater than 0.86, greater than 0.87, greater than 0.88, greater than 0.89, greater than 0.90, greater than 0.91, greater than 0.92, greater than 0.93, greater than 0.94, greater than 0.95, greater than 0.96, greater than 0.97, greater than 0.98, or greater than 0.99 when matching or aligning the one or more TATA box motif(s) to TATA box consensus, and one or more Y patch promoter element(s) of the donor promoter.

In yet another embodiment, the promoter activating nucleic acid sequence described in any of the embodiments above has a sequence identity of at least 75%, 80%, 85%, 90%, 95%, 96%, 97%, 98%, or 99% to or is identical to one of the sequences of SEQ ID NOs: 1 to 30, GTATAAAAG (E59), CTATAAATA (E59a), CTATATATA (E59b), CTATAAAAA (E59c) and CTATATAAA (E59d), preferably over the whole length of the promoter activating nucleic acid sequence, preferably SEQ ID NO: 1, SEQ ID NO: 2, SEQ ID NO: 9, SEQ ID NO: 10, SEQ ID NO: 11, SEQ ID NO: 12, SEQ ID NO: 13, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d), particularly preferably SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d).

In one embodiment, there is provided a promoter activating nucleic acid sequence as described in any of the embodiments above, wherein the cell or organism is a plant cell or plant.

In another embodiment, the recipient promoter and/or the donor promoter is/are a plant promoter.

In a further embodiment, the recipient promoter and the donor promoter are different and/or originate from the same species or from different species.

In one embodiment, the plant or plant cell or plant promoter described in any of the embodiments above, originates from a genus selected from the group consisting of *Hordeum, Sorghum, Saccharum,* Zea, *Setaria, Oryza, Triticum, Secale, Triticale, Malus, Brachypodium, Aegilops, Daucus, Beta, Eucalyptus, Nicotiana, Solanum,* Coffea, *Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell or plant promoter originates from a species selected from the group consisting of *Hordeum vulgare, Hordeum bulbusom, Sorghum bicolor, Saccharum officinarium,* Zea *spp., including* Zea *mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum,* Coffea *canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum,* Ca*janus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In another embodiment, upon site-specific insertion or introduction of the promoter activating nucleic acid sequence into the recipient promoter, the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the insertion or introduction.

In another aspect, the present invention provides a chimeric promoter comprising a recipient promoter or the core promoter thereof and at least one promoter activating nucleic acid sequence as described in any of the embodiments above inserted or introduced at a position upstream or downstream of the transcription start site of the recipient promoter.

In one embodiment, a chimeric promoter as described above is provided, wherein the promoter activating nucleic acid sequence is inserted or introduced by addition and/or deletion and/or substitution of one or more nucleotides into the recipient promoter at a position
i. 500 nucleotides or less, preferably 150 nucleotides or less upstream of the transcription start site, and/or
ii. 50 or more nucleotides upstream of the start codon; and/or
iii. where there is no upstream open reading frame (uORF) downstream of the insertion or introduction site.

In a further aspect, the present invention provides a delivery system comprising the promoter activating nucleic acid sequence and/or the chimeric promoter as described in any of the embodiments above, and/or means for site-specific insertion or introduction of the promoter activating nucleic acid sequence described above into a recipient promoter.

In yet another aspect, the present invention provides a nucleic acid construct or an expression cassette comprising the promoter activating nucleic acid sequence as described in any of the embodiments above and/or the chimeric promoter as described in any of the embodiments above.

In a further aspect, the present invention also provides a vector comprising the promoter activating nucleic acid sequence as described in any of the embodiments above, and/or the chimeric promoter as described in any of the embodiments above or the nucleic acid construct and/or the expression cassette as described above, and/or means for site-specific insertion or introduction of the promoter activating nucleic acid sequence described above into a recipient promoter.

In another aspect, the present invention provides a cell or organism or a progeny thereof or a part of the organism or progeny thereof,
a) in which a promoter activating nucleic acid as described in any of the embodiments above is inserted or introduced by addition and/or deletion and/or substitution of one or more nucleotides into a recipient promoter controlling the expression of a nucleic acid molecule of interest in the cell or the organism, preferably inserted or introduced at a position upstream or downstream of the transcription start site of the recipient promoter, more preferably introduced at a position
   i. 500 nucleotides or less, preferably 150 nucleotides or less upstream of the transcription start site of the nucleic acid molecule of interest, and/or
   ii. 50 or more nucleotides upstream of the start codon of the nucleic acid molecule of interest; and/or
   iii. where there is no upstream open reading frame (uORF) downstream of the insertion site, or
b) comprising the chimeric promoter as described in any of the embodiments above, the delivery system as described in any of the embodiments above, the nucleic acid construct or an expression cassette as described above or the vector as described above.

In one embodiment, in the cell or organism or the progeny thereof or the part of the organism or the progeny thererof as described above, the recipient promoter is a plant promoter.

In another embodiment, in the cell or organism or the progeny thereof or the part of the organism or the progeny thererof as described above, the nucleic acid molecule of interest is heterologous or native to the recipient promoter and/or is an endogenous or exogenous nucleic acid molecule to the cell or organism.

In a further embodiment, the cell or organism or the progeny thereof or the part of the organism or the progeny thererof according to any of the embodiments described above, is a plant cell or plant or part thereof, preferably wherein the plant originates from a genus selected from the group consisting of *Hordeum, Sorghum, Saccharum,* Zea, *Setaria, Oryza, Triticum, Secale, Triticale, Malus, Brachypodium, Aegilops, Daucus, Beta, Eucalyptus, Nicotiana, Solanum,* Coffea, *Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell originates from a species selected from the group consisting of *Hordeum vulgare, Hordeum bulbusom, Sorghum bicolor, Saccharum officinarium,* Zea *spp., including* Zea *mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum,* Coffea *canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In a further aspect, the present invention provides a method for identifying a promoter activating nucleic acid sequence or a chimeric promoter, comprising:
i) identifying a gene in a cell or an organism having a high expression level,
ii) isolating one or more contiguous stretch(es) from the promoter of the gene identified in step i) wherein the one or more contiguous stretch(es) originate(s) a) from the core promoter of the said donor promoter or b) from a sequence from position -50 to position +20 relative to the transcription start site of said donor promoter,
iii) inserting or introducing by addition and/or deletion and/or substitution of one or more nucleotides the one or more contiguous stretch(es) into a recipient promoter controlling the expression of a nucleic acid molecule of interest at a position upstream or downstream of the transcription start site of the recipient promoter,
iv) determining in a cell or organism or *in vitro* the expression level of the nucleic acid molecule of interest under the control of the recipient promoter comprising the insertion or introduction of step iii) relative to the expression level of the same or another nucleic acid molecule of interest under the control of the recipient promoter without the insertion or introduction of step iii) or to another reference promoter in a given environment and/or under given genomic and/or environmental conditions, wherein the nucleic acid molecule of interest is heterologous or native to the recipient promoter and/or is endogenous or exogenous to the cell or organism, and
v) identifying and thus providing the promoter activating nucleic acid sequence as described in any of the embodiments above or the chimeric promoter as described in any of the embodiments above when increased expression of the nucleic acid molecule of interest in step iv) is observed,
vi) optionally, shortening the promoter activating nucleic acid sequence identified in step v) stepwise and repeating steps iv) and v) at least one time and/or modifying one or more TATA box motif(s) present in the promoter activating nucleic acid sequence identified in step v) or in the recipient promoter by addition and/or substitution and/or deletion of one or more nucleotides for converting the one or more TATA box motif(s) into one or more TATA box motif(s) having increased or higher relative score(s) when matching or aligning the one or more TATA box motif(s) to the TATA box consensus, and repeating steps iv) and v) at least one time.

In one embodiment of the method described above, in step iii) the one or more contiguous stretch(es) is/are inserted or introduced into the recipient promoter at a position
(a) 500 nucleotides or less, preferably 150 nucleotides or less upstream of the transcription start site of the nucleic acid molecule of interest; and/or
(b) more than 50 nucleotides upstream of the start codon of the nucleic acid molecule of interest; and/or
(c) where there is no upstream open reading frame (uORF) downstream of the insertion or introduction site.

In yet another aspect, the present invention provides a method for increasing the expression level of a nucleic acid molecule of interest in a cell, comprising:
ia) introducing into the cell the promoter activating nucleic acid sequence as described in any of the embodiments above, the chimeric promoter as described above, the delivery system as described in any of the embodiments above, or the nucleic acid construct or an expression cassette as described above; or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any of the embodiments above into a recipient promoter controlling the expression of the nucleic acid molecule of interest in the cell at a position upstream or downstream of the transcription start site of the recipient promoter controlling the expression of the nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of the nucleic acid molecule of interest by addition and/or deletion and/or substitution so that a promoter activating nucleic acid sequence as defined in any of the embodiments above is formed, and
iiic) optionally, modifying one or more TATA box motif(s) present in the promoter activating nucleic acid sequence inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus.

In a further aspect, the present invention provides a method for producing a cell or organism having an increased expression level of a nucleic acid molecule of interest, comprising:
ia) introducing into the cell the promoter activating nucleic acid sequence according to any of the embodiments described above, the chimeric promoter as described above, the delivery system as described above, or the nucleic acid construct or an expression cassette as described above, or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any of the embodiments above or the chimeric promoter as defined in any of the embodiments above into a recipient promoter controlling the expression of the nucleic acid molecule of interest in the cell at a position upstream or downstream of the transcription start site of the recipient promoter controlling the expression of a nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of a nucleic acid molecule of interest by addition and/or deletion and/or substitution so that a promoter activating nucleic acid sequence as defined in any of the embodiments above is formed, and
iiic) optionally, modifying one or more TATA box motif(s) present in the promoter activating nucleic acid sequence or the chimeric promoter inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus, and
iv) obtaining a cell or organism having increased expression level of a nucleic acid molecule of interest upon insertion of the promoter activating nucleic acid sequence as defined in any of the embodiments above or upon modification to form the promoter activating nucleic acid sequence as defined in any of the embodiments above.

In another aspect, the present invention provides a method for producing a transgenic cell or transgenic organism having increased expression level of a nucleic acid molecule of interest, comprising:
i) transforming or transfecting a cell with the promoter activating nucleic acid sequence as described in any of the embodiments above, the chimeric promoter as described above, the delivery system as described above, or the nucleic acid construct or an expression cassette as described above, or the vector as described above; and
ii) optionally, regenerating a transgenic organism from the transgenic cell or a transgenic progeny thereof.

In one embodiment of the methods described above, the cell or organism is a plant cell or plant or a progeny thereof, preferably wherein the plant originates from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In another embodiment of the methods as described above, the nucleic acid molecule of interest is selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

In yet another aspect, the present invention provides a cell or organism or a progeny thereof, preferably a plant cell or plant or progeny thereof, obtainable by a method as described above.

In one aspect, the present invention also relates to the use of the promoter activating nucleic acid sequence as described in any of the embodiments above, the delivery system as described above, the nucleic acid construct or the expression cassette as described above or the vector as described above for increasing the expression level of a nucleic acid molecule of interest in a cell or organism upon site-specific insertion or introduction into a recipient promoter controlling the expression of the nucleic acid molecule of interest.

Further aspects and embodiments of the present invention can be derived from the subsequent detailed description, the drawings, the sequence listing as well as the attached set of claims.

### Brief description of the Drawings

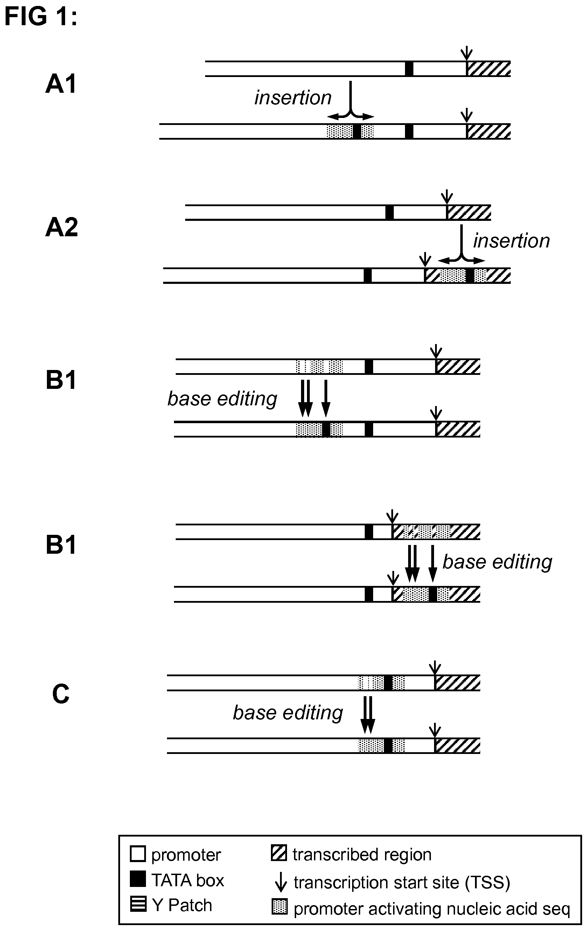

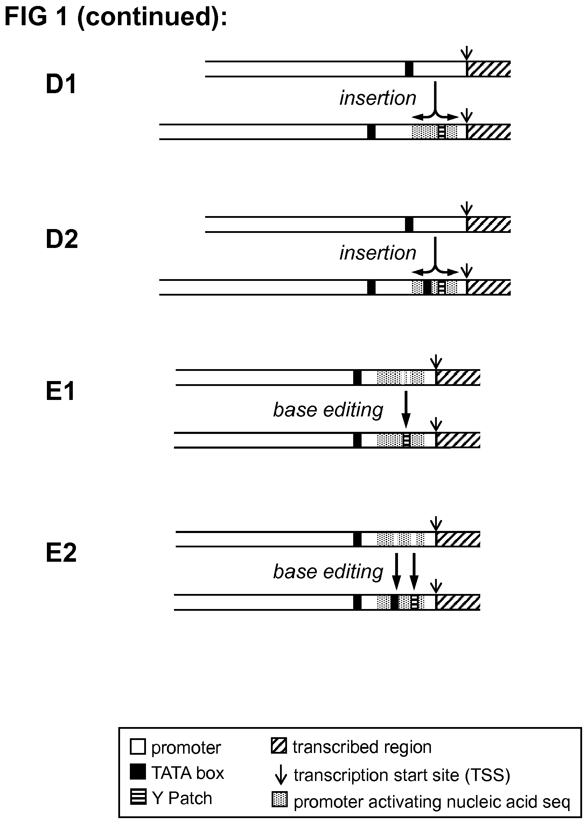

**Figure 1** shows exemplary possible approaches to introduce the promoter activating nucleic acid sequences of the present invention into a recipient promoter. **A1:** a promoter activating sequence comprising a TATA box motif is inserted into the recipient promoter upstream of the core promoter. **A2:** a promoter activating sequence comprising a TATA box motif is inserted into the recipient promoter downstream of TSS. **B1:** a promoter activating sequence comprising a TATA box motif is introduced by base editing of nucleotides of the recipient promoter upstream of the core promoter. **B2:** a promoter activating sequence comprising a TATA box motif is introduced by base editing of nucleotides of the recipient promoter downstream of TSS. **C:** the core promoter of the recipient is modified by base editing to form an activating sequence. **D1:** a promoter activating sequence comprising a Y patch is inserted into the recipient promoter downstream of the core promoter. **D2:** a promoter activating sequence comprising a TATA box motif and a Y patch is inserted into the recipient promoter downstream of the core promoter. **E1:** a promoter activating sequence comprising a Y patch is introduced by base editing of nucleotides of the recipient promoter downstream of the core promoter. **E2:** a promoter activating sequence comprising a TATA box motif and a Y patch is introduced by base editing of nucleotides of the recipient promoter downstream of the core promoter.
**Figure 2** shows a schematic overview of the strategy for identification and testing of ≤ 20 bp DNA elements for activation of expression.
**Figure 3** shows the vector used in the transient expression assay described in example 1.
**Figure 4** shows the activation of the target gene promoter Zm-prom1 by insertion of the DNA elements E53, E55, E56, E61, E62, E63, E64, E65, E66, E67, E68, E69, E70 and E71. The promoter activity is quantified with respect to the activity of the unmodified promoter Zm-prom 1, which therefore has an activity of 1.
**Figure 5** shows the activation of the target gene promoters Zm-prom1, Zm-prom2 and Zm-prom3 by insertion of the DNA elements E53b. The promoter activity is quantified with respect to the activity of the respective unmodified promoters, which therefore have an activity of 1.
**Figure 6** shows the construct pKWS399_35S:Luci_Zm-prom1:NLuc used for the transformation of corn in example 2.
**Figure 7** shows the construct pKWS399_35S:Luci_Zm-prom1+E55a:NLuc used for the transformation of corn in example 2.
**Figure 8** shows the construct pKWS399_35S:Luci_Zm-prom1:Zm1-genomic used for the transformation of corn in example 3.
**Figure 9** shows the construct pKWS399_35S:Luci_Zm-prom1+E55a:Zm1-genomic used for the transformation of corn in example 3.

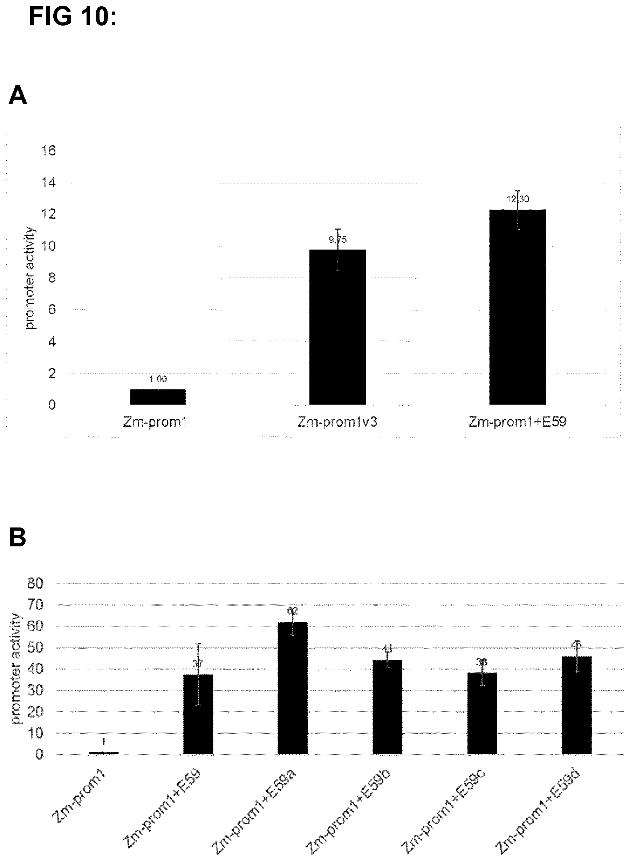

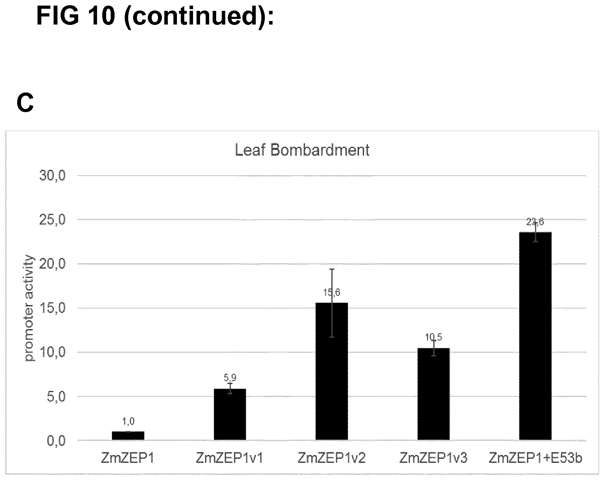

**Figure 10** shows in **A** the activation of the target gene Zm-prom1 by conversion to E59 (Zm-prom1v3) and by insertion of E59 further downstream of the original TSS (Zm-prom1+E59) as determined in example 6. The promoter activity is quantified with respect to the activity of the unmodified promoter Zm-prom1, which therefore has an activity of 1. **B**: Optimization of element E59 by aligning to TATA box consensus. The activating effect is measured in a transient assay system based on corn leaf bombardment with respective promoter-reporter constructs followed by luciferase measurement. **C:** Original TATA-box of a promoter of interest (e.g ZmZEP1) has been modified by means of base editing (ZmZEP1v1, ZmZEP1v2 and ZmZEP1v3) according to identified DNA segment E59, E53f, E55a, respectively. Additionally, the element E53b has been inserted into ZmZEP1. The activating effect is measured in a transient assay system based on corn leaf bombardment with respective promoter-reporter constructs followed by luciferase measurement.
**Figure 11** shows nucleotide frequencies matrices and motif logos for TATA box consensus of vertebrates **(A)** (derived from http://jaspar.genereg.net/matrix/MA0108.1/), dicotyledonous plants **(B)** and monocotyledonous plants **(C)** (Shahmuradov et al. (2003). PlantProm: a database of plant promoter sequences. Nucleic acids research, 31(1), 114-117.; http://linux1.softberry.com/berry.phtml/freedownloadhelp/viewers/gmv/berry.phtml ?topic=plantprom&group=data&subgroup=plantprom).

### Definitions

A "promoter" refers to a DNA sequence capable of controlling and/or regulating expression of a coding sequence, i.e., a gene or part thereof, or of a functional RNA, i.e. a RNA which is active without being translated, for example, a miRNA, a siRNA, an inverted repeat RNA or a hairpin forming RNA. A promoter is usually located at the 5' part of a gene. Promoters can have a broad spectrum of activity, but they can also have tissue or developmental stage specific activity. For example, they can be active in cells of roots, seeds and meristematic cells, etc. A promoter can be active in a constitutive way, or it can be inducible. The induction can be stimulated by a variety of environmental conditions and stimuli. Often promoters are highly regulated. A promoter of the present disclosure may include an endogenous promoter natively present in a cell, or an artificial or transgenic promoter, either from another species, or an artificial or chimeric promoter, i.e. a promoter that does not naturally occur in nature in this composition and is composed of different promoter elements. The process of transcription begins with the RNA polymerase (RNAP) binding to DNA in the promoter region, which is in the immediate vicinity of the transcription start site (TSS) at the position +1. From analysis in Arabidopsis thaliana the most frequently observed sequence at this position is CA, and TA was the second. There is a strong preference of a dimer sequence at the -1/+1 position. It has been clearly shown that most of the TSS is A or G, and the -1 position is likely to be C or T. This YR Rule (Y: C or T, R: A or G) applies to as many as 77% of the Arabidopsis promoters that is a much higher frequency than expected random appearance (25%) (Yamamoto et al. (2007). Identification of plant promoter constituents by analysis of local distribution of short sequences. BMC genomics, 8(1), 67.). A typical promoter sequence is thought to comprise some regulatory sequence motifs positioned at specific sites relative to the TSS. These cis-regualtory elements are e.g. binding sites for transacting factors such as transcription factors. The structure of eukaryotic promoters may be rather complex as they have several different sequence motifs, such as TATA box, INR box, BRE, CCAAT-box and GC-box (Bucher P., J. Mol. Biol. 1990 Apr 20; 212(4):563-78.) or Y Patch promoter elements (Yamamoto et al. (2007). Identification of plant promoter constituents by analysis of local distribution of short sequences. BMC genomics, 8(1), 67; Civáň, P., & Svec, M. (2009). Genome-wide analysis of rice (Oryza sativa L. subsp. japonica) TATA box and Y Patch promoter elements. Genome, 52(3), 294-297.). Promoters can be of varying length and may span more than a thousand nucleotides. Finally, promoter architectures and function differ in different taxa. In particular, there are huge differences between eukaryotic and prokaryotic promoters, but also eukaryotic promoters, e.g., plant promoters and mammalian cell promoters, differ in structure, function and the regulatory network within the cell. Therefore, findings derived from studies with mammalian cell promoters may not necessarily apply for plant promoters within a plant cell environment.

A "core promoter" generally refers to the region of the promoter that is necessary to initiate the transcription and includes at least the preinitiation complex binding site. It is less than 100 nucleotides long and spans approximately positions -45 to +15 relative to the transcription start site.

A "donor promoter" refers to a native promoter found in a certain cell or organism, which comprises a core promoter sequence that enables a high expression of the nucleic acid molecule, which is under the control of the promoter. The core promoter or one or more continuous stretch(es) of the core promoter can be identified, which upon site-specific introduction, e.g. insertion, to a "recipient promoter" increase the expression of the nucleic acid molecule under the control of the recipient promoter.

A "recipient promoter" is therefore a promoter, which can be modified by introduction of a core promoter sequence or one or more continuous stretch(es) of the core promoter of a donor promoter leading to increased expression of the nucleic acid molecule under the control of the recipient promoter.

A "promoter activating nucleic acid sequence" refers to a nucleic acid sequence or to one more contiguous stretch(es) of nucleotides, which upon site-specific introduction, e.g. insertion, into a promoter increase(es) the expression of the nucleic acid molecule under the control of the promoter

A "chimeric promoter" is a promoter that does not exist in nature in its specific configuration. As used herein it refers to a promoter comprising one or more nucleotide sequences of both, a donor and a recipient promoter. The term refers in particular to a recipient promoter or the core promoter of the recipient promoter comprising one or more promoter activating nucleic acid sequence(s) or one or more contiguous stretch(es) representing a promoter activating nucleic acid sequence introduced into the recipient promoter sequence at one or more specific sites. The introduction of the one or more promoter activating nucleic acid sequence(s) may be achieved by insertion into or by modification of the sequence of the recipient promoter, i.e. addition of one or more contiguous or non-contiguous nucleotide(s) to the recipient promoter sequence, or substitution or deletion of one or more contiguous or non-contiguous nucleotide(s) of the recipient promoter sequence.

A nucleic acid sequence "configured for site-specific insertion" refers to a nucleic acid sequence, which has been taken out of its genomic context, i.e. it does not comprise neighbouring or flanking regions or part of a chromosome as found in the cell or organism that it is endogenous to but it is provided as part of a delivery system, an insertion construct or an expression construct, which can be employed by known techniques for insertion, into a specific site of a given promoter sequence. It can be a synthetic or biological sequence or comprise parts of both.

"Introducing" a nucleic acid or "introduction" of a nucleic acid or a nucleic acid sequence into a second nucleic acid or nucleic acid sequence refers to any modification of the second nucleic acid that results in the presence of the nucleic acid sequence of the first nucleic acid within the nucleic acid sequence of the second nucleic acid, where it has not been present previous to the modification. In particular, such a modification can be achieved by addition, substution or detetion of one or more nucleotides or any combination of these.

"Modifying a (nucleic acid) sequence" in the context of the present invention refers to any change of a (nucleic acid) sequence that results in at least one difference in the (nucleic acid) sequence distinguishing it from the original sequence. In particular, a modification can be achived by insertion or addition of one ore more nucleotide(s), or substitution or deletion of one ore more nucleotide(s) of the original sequence or any combination of these.

"Addition" refers to one or more nucleotides being added to a nuceic acid sequence, which may be contiguous or single nucleotides added at one or more positions within the nucleic acid sequence.

"Substitution" refers to the exchange of one or more nucleotide(s) of a nucleic acid sequence by one or more different nucleotide(s). A substitution may be a replacement of one or more nucleotide(s) or a modification of one or more nucleotide(s) that results in (a) different nucleotide(s) e.g by conversion of a nucleobase to a different nucleobase.

"Deletion" refers to the removal of one or more nucleotide(s) from a nucleic acid sequence.

The term "heterologous" herein refers to an element such as a nucleic acid that has been transferred to a context, i.e. an organism or a genomic location, in which it does not naturally occur. A nucleic acid molecule that is heterologous to a certain promoter therefore refers to a nucleic acid molecule that is not naturally found within this promoter. On the other hand, the term "native" means that an element such as a nucleic acid is present in a context, i.e. an organism or a genomic location, in which it naturally occurs. Accordingly, a nucleic acid molecule that is native to a certain promoter therefore refers to a nucleic acid molecule that is naturally found to be structural part of this of this promoter.

A nucleic acid molecule that is "endogenous" to a cell or organism refers to a nucleic acid molecule that naturally occurs in the genome of this cell or organism. On the other hand, a nucleic acid molecule that is "exogenous" to a cell or organism refers to a nucleic acid molecule that does not naturally occur in this cell or organism but has been inserted or introduced.

A "gene" as used herein refers to a DNA region encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

The term "gene expression" or "expression" as used herein refers to the conversion of the information, contained in a gene or nucleic acid molecule, into a "gene product" or "expression product". A "gene product" or "expression product" can be the direct transcriptional product of a gene or nucleic acid molecule (e.g., mRNA, tRNA, rRNA, antisense RNA, ribozyme, structural RNA or any other type of RNA) or a protein produced by translation of an mRNA. Gene products or expression products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

An "increased expression of the nucleic molecule of interest" is observed, when the expression of the nucleic molecule of interest is higher when the promoter controlling the expression has been modified by introducing, e.g. inserting, the promoter activating sequence at a specific position compared to the expression of the same nucleic molecule of interest under the control of the same promoter without the modification, e.g. the insertion. "increased expression of the nucleic molecule of interest" means that the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the inserted or introduced promoter activating nucleic acid sequence.

A "high expression level" as used herein refers to an expression level comparable to the expression levels of the about 250 most active genes such as the S-adenosyl methionine decarboxylase 2 (SAM2, GRMZM2G154397). Preferably, genes with a high expression level according to the present disclosure have average FPKM values of >1000 in different tissues or under different genomic and/or environmental conditions. FPKM (Fragments Per Kilobase of transcript per Million mapped reads) refers to sequenced fragments of transcripts normalized by dividing by the total length of the transcript. This results in the metric fragments per kilobase of transcript per million mapped reads.

"Environment" or "environmental conditions" refer to external conditions such as nutrient concentrations, temperature or pH that a cell or tissue is exposed to. A "genomic condition" refers to internal conditions that a cell or tissue experiences such as a developmental stage, a cell division or differentiation stage.

A "reference gene" refers to a gene the expression level of which is used as a reference to assess the expression level of a gene of interest. Suitable reference genes are genes the expression levels of which do not vary much but remain constant under any given environmental or genomic conditions.

A "contiguous stretch" refers to a nucleic acid sequence, i.e. a specific sequential order of nucleotides that occurs e.g. in a promoter. One donor promoter can harbour several contiguous stretches that form a promoter activating nucleic acid sequence, and which may or may not neighbour each other and which may or may not fully or partially overlap each other. Typically, each contiguous stretch comprises at least 6, at least 7, at least 8, at least 9 or at least 10 nucleotides, or six or more nucleotides.

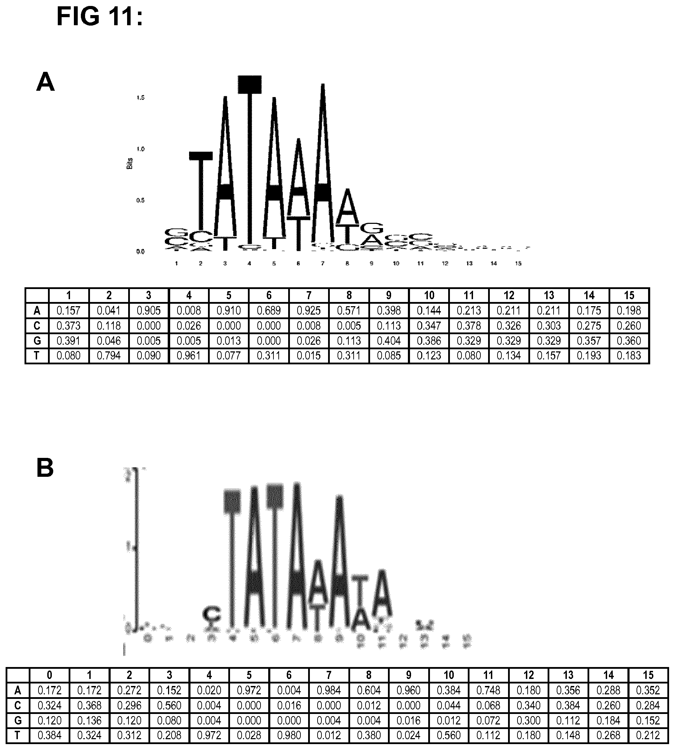

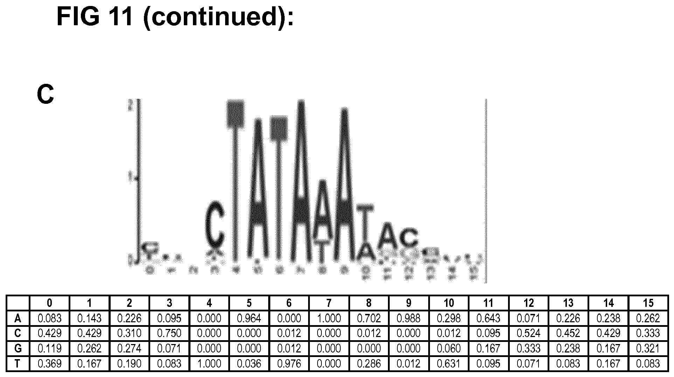

A "TATA box motif" refers to a sequence found in many core promoter regions of eukaryotes. The TATA-box motif is usually found within 100 nucleotides upstream of the transcription start site. It generally contains the consensus sequence 5'-TATA(A/T)A(A/T)-3. Preferably, it contains a sequence exhibiting a relative score of greater than 0.8 when matching or aligning the sequence found in the core promoter region to a TATA box consensus as defined further below. Preferably the relative score is greater than 0.85 or greater than 0.9, more preferably greater than 0.95, greater than 0.96, greater than 0.97, greater than 0.98 or greater than 0.99. For scoring a DNA input sequence comprising the TATA-box motif, the sequence can be analyzed by software tools (e.g. http://jaspar.genereg.net/ (Mathelier, A., Zhao, X., Zhang, A. W., Parcy, F., Worsley-Hunt, R., Arenillas, D. J., ... & Lim, J. (2013). JASPAR 2014: an extensively expanded and updated open-access database of transcription factor binding profiles. Nucleic acids research, 42(D1), D142-D147.)). Hereby, a score is computed using the pssm.search function available in BioPython (http://biopython.org/DIST/docs/api/Bio.motifs.matrix.PositionSpecificScoringMatri x-class.html#search). The relative score is a threshold score in the range 0 to 1, which is computed as follows: *relative_score* = *(score* - *min_score)* / *(max_score* - *min_score)* (http://biopython.org/DIST/docs/tutorial/Tutorial.html#htoc214). Depending on the origin of the DNA input sequence different "TATA box consensus" are to be used for scoring. "TATA box consensus" for vertebrates, dicotyledonous plants and monocotyledonous plants are defined in Figure 11 as nucleotide frequencies matrices and motif logos. TATA-box motifs in the analyzed 60 bp candidates. A relative score of 1 indicates a perfect TATA box consensus while a relative score ≤ 0.8 indicates that no TATA box is present.

A "Y patch promoter element" or "pyrimidine patch promoter element" or "Y patch" or "pyrimidine patch" refers to a sequence found in many promoters of higher plants. A typical Y patch is composed of C and T (pyrimidine) (Yamamoto et al. (2007). Identification of plant promoter constituents by analysis of local distribution of short sequences. BMC genomics, 8(1), 67.). Y patch can be detected by our LDSS analysis as well as by a search for consensus sequence from plant promotors, preferably core promoters, by MEME and AlignACE (ex-amplary motif for A. thaliana: TTTCTTCTTC) (Molina & Grotewold. Genome wide analysis of Arabidopsis core promoters. BMC Genomics. 2005;6:25.). The Y patch is usually found within 100 nucleotides upstream of the transcription start site (postion -1 to -100 relative to TSS), preferably at a position between -10 to - 60 relative to TSS.

The "transcription start site" refers to the first nucleotide of a DNA sequence that is transcribed. This nucleotide is assigned the position +1 within the promoter.

"Upstream" and "downstream" relate to the 5' to 3' direction in which RNA transcription takes place. Upstream is toward the 5' end of the RNA molecule and downstream is toward the 3' end. On the DNA from which transcription takes plase, upstream is toward the 5' end of the coding strand for the gene in question and downstream is toward the 3' end.

An "upstream open reading frame (uORF)" refers to an open reading frame located upstream of the initiation codon of a main coding region. uORFs are usually involved in the regulation of the expression of the main coding region downstream.

The term "one ore more" includes "one or two", "one, two or three", "one, two, three or four" and "one, two, three, four or five", but has generally the meaning of "at least one". As an example, one or more TATA box motif(s) may be one or two TATA box motif(s), one, two or three TATA box motif(s), one, two, three or four TATA box motif(s), one, two, three, four or five TATA box motif(s), or at least one TATA box motif.

The terms "plant" or "plant cell" as used herein refer to a plant organism, a plant organ, differentiated and undifferentiated plant tissues, plant cells, seeds, and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. The cells can have any degree of ploidity, i.e. they may either be haploid, diploid, tetraploid, hexaploid or polyploid.

The term "progeny" as used herein in the context of a eukaryotic cell, preferably an animal cell and more preferably a plant or plant cell or plant material according to the present disclosure relates to the descendants of such a cell or material which result from natural reproductive propagation including sexual and asexual propagation. It is well known to the person having skill in the art that said propagation can lead to the introduction of mutations into the genome of an organism resulting from natural phenomena which results in a descendant or progeny, which is genomically different to the parental organism or cell, however, still belongs to the same genus/species and possesses mostly the same characteristics as the parental recombinant host cell. Such progeny resulting from natural phenomena during reproduction or regeneration are thus comprised by the term of the present disclosure and can be readily identified by the skilled person when comparing the "progeny" to the respective parent or ancestor.

The terms "delivery system", "nucleic acid construct", "expression cassette" or "vector" refer to elements used for introducing at least one nucleic acid sequence into a cellular system. The nucleic acid sequence to be introduced may be targeted for site-specific insertion into genomic DNA and/or transient expression in the cellular system. Alternatively, it may be introduced by modifications such as addition, deletion or substitution of one or more nucleotides of a sequence, e.g. a genomic sequence, which is present in the target cell or cellular system. The introduction may also be achieved by any combination of the above modifications, i.e. insertion, addition deletion and substitution. The elements *inter alia* comprise one or more plasmid(s) or (plasmid) vector(s), cosmid(s), artificial yeast- or bacterial artificial chromosome(s) (YACs and BACs), phagemide(s), bacterial phage based vector(s), isolated single-stranded or double-stranded nucleic acid sequence(s), comprising DNA and RNA sequences in linear or circular form, or amino acid sequences, viral vector(s), including modified viruses, and a combination or a mixture thereof, for introduction or transformation, transfection or transduction into any prokaryotic or eukaryotic target cell, including a plant, plant cell, tissue, organ or material according to the present disclosure. Any of the elements may be carrying the nucleic acid construct(s) or expression cassette(s) and/or tools for site-specific introduction.

A "means for site-specific modification of a nucleic acid sequence" and, more specifically, a "means for site-specific introduction of a promoter activating nucleic acid sequence" refers to any tool required to achive a modification of a recipient promoter sequence, so that a promoter activating sequence as described herein is formed, which has previously not been present. Such means include any tools for site-specific modification, i.e. insertion, addition, substitution or deletion, of nucleic acid sequences known to the skilled person. Examples are, in particular, "site-specific effectors" such as nucleases, nickases, recombinases, transposases, base editors or molecular complexes including these tools. These effectors have the capacity to introduce a single- or double-strand cleavage into a genomic target site, or have the capacity to introduce a targeted modification, including a point mutation, an insertion, or a deletion, into a genomic target site of interest. A site-specific effector can act on its own, or in combination with other molecules as part of a molecular complex. The site-specific effector can be present as fusion molecule, or as individual molecules associating by or being associated by at least one of a covalent or non-covalent interaction so that the components of the site-specific effector complex are brought into close physical proximity. The complex may include a repair template to make a targeted sequence conversion or replacement at the target site. A repair template (RT) represents a single-stranded or double-stranded nucleic acid sequence, which can be provided during any genome editing causing a double-strand or single-strand DNA break to assist the targeted repair of said DNA break by providing a RT as template of known sequence assisting homology-directed repair.

A "site-specific nuclease" refers to a nuclease or an active fragment thereof, which is capable to specifically recognize and cleave DNA at a certain location. This location is herein also referred to as a "predetermined location". Such nucleases typically produce a double strand break (DSB), which is then repaired by nonhomologous end-joining (NHEJ) or homologous recombination (HR). The nucleases include zinc-finger nucleases, transcription activator-like effector nucleases, CRISPR/Cas systems, including CRISPR/Cas9 systems, CRISPR/Cpf1 systems, CRISPR/C2C2 systems, CRISPR/CasX systems, CRISPR/CasY systems, CRISPR/Cmr systems, engineered homing endonucleases, recombinases, transposases and meganucleases, and/or any combination, variant, or catalytically active fragment thereof.

A "CRISPR nuclease", as used herein, is any nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. Said DNA recognition can be PAM (protospacer adjacent motif) dependent. CRISPR nucleases having optimized and engineered PAM recognition patterns can be used and created for a specific application. The expansion of the PAM recognition code can be suitable to target site-specific effector complexes to a target site of interest, independent of the original PAM specificity of the wild-type CRISPR-based nuclease. Cpf1 variants can comprise at least one of a S542R, K548V, N552R, or K607R mutation, preferably mutation S542R/K607R or S542R/K548V/N552R in AsCpf1 from *Acidaminococcus.* Furthermore, modified Cas or Cpf1 variants or any other modified CRISPR effector variants, e.g., Cas9 variants, can be used according to the methods of the present invention as part of a base editing complex, e.g. BE3, VQR-BE3, EQR-BE3, VRER-BE3, SaBE3, SaKKH-BE3 (see Kim et al., Nat. Biotech., 2017, doi:10.1038/nbt.3803). Therefore, according to the present invention, artificially modified CRISPR nucleases are envisaged, which might indeed not be any "nucleases" in the sense of double-strand cleaving enzymes, but which are nickases or nuclease-dead variants, which still have inherent DNA recognition and thus binding ability. Suitable Cpf1-based effectors for use in the methods of the present invention are derived from *Lachnospiraceae bacterium* (LbCpf1, e.g., NCBI Reference Sequence: WP_051666128.1), or from *Francisella tularensis* (FnCpf1, e.g., UniProtKB/Swiss-Prot: A0Q7Q2.1). Variants of Cpf1 are known (cf. Gao et al., BioRxiv, dx.doi.org/10.1101/091611). Variants of AsCpf1 with the mutations S542R/K607R and S542R/K548V/N552R that can cleave target sites with TYCV/CCCC and TATV PAMs, respectively, with enhanced activities *in vitro* and *in vivo* are thus envisaged as site-specific effectors according to the present invention. Genome-wide assessment of off-target activity indicated that these variants retain a high level of DNA targeting specificity, which can be further improved by introducing mutations in non-PAM-interacting domains. Together, these variants increase the targeting range of AsCpf1 to one cleavage site for every -8.7 bp in non-repetitive regions of the human genome, providing a useful addition to the CRISPR/Cas genome engineering toolbox (see Gao et al., supra).

A "base editor" as used herein refers to a protein or a fragment thereof having the same catalytical activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest. Preferably, the at least one base editor in the context of the present invention is temporarily or permanently linked to at least one site-specific effector, or optionally to a component of at least one site-specific effector complex. The linkage can be covalent and/or non-covalent.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/ emboss_water/nucleotide.html) nucleic acids or the EMBOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

### Detailed Description

The present invention relates to several aspects to establish a new technology to increase the expression of endogenous or exogenous nucleic acid molecules up to many times by inserting or introducing promoter activating sequences into the promoter controlling the expression of endogenous or exogenous nucleic acid molecules.

In a first aspect, a promoter activating nucleic acid sequence is provided, which is configured for targeted site-specific insertion into a recipient promoter controlling the expression of a nucleic acid molecule of interest in a cell or an organism, wherein the promoter activating nucleic acid sequence causes an increased expression of the nucleic acid molecule of interest upon site-specific insertion, preferably wherein the nucleic acid molecule of interest is heterologous or native to the recipient promoter and/or is an endogenous or exogenous nucleic acid molecule to the cell or organism.

The promoter activating nucleic acid sequences provided by the present invention can be broadly applied to increase the expression of any nucleic acid molecule of interest in a cellular context. The promoter activating nucleic acid sequences are usually double stranded DNA molecules that can be inserted into the promoter controlling the expression of the nucleic acid molecule of interest (Figure 1 A1, A2, D1 and D2).

The application is not limited to certain promoters or nucleic acid molecules of interest or combinations of both. In one embodiment the nucleic acid molecule of interest is endogenous to the cell or organism that it is expressed in. In this case the promoter to be activated may be the promoter that natively controls the expression of this nucleic acid molecule of interest in the cell or organism but it is also possible that the endogenous nucleic acid molecule of interest is under the control of a heterologous promoter, which does not natively control its expression. Alternatively, the nucleic acid molecule of interest is exogenous to the cell or organism that it is expressed in. In this case the promoter may also be exogenous to the cell or organism but it may be the promoter that the nucleic acid molecule of interest is controlled by in its native cellular environment. On the other hand, the promoter may also be exogenous to the cell or organism in that it is to be activated and at the same time be hererologous to the nucleic acid molecule of interest.

The present invention thus provides a technical guidance to first identify a promoter to be optimized. Next, it is taught how said promoter can be modified in the most suitable way in a targeted manner based on the findings on promoter activating nucleic acid molecules presented herein. Furthermore, strategies to implement the modification are presented.

In one embodiment, the promoter activating nucleic acid sequence as described above has a length between 6 and 70 nucleotides, preferably between 7 and 60 nucleotides, more preferably between 8 and 40 nucleotides and most preferably between 9 and 20 nucleotides.

The promoter activating nucleic acid sequence provided by the present invention may represent the core promoter region of a donor promoter that has been found to have a high activity, i.e. a high level of gene expression, in most tissues and under most conditions. However, as demonstrated in the following description, such core promoter regions of around 60 nucleotides length may be significantly shortened without loosing their activating properties. Sequences of 20 nucleotides and less have been found to be capable to increase recipient promoter activity by many fold upon insertion or introduction. Thus, minimal modifications of the sequence of the recipient promoter can result in a significantly increased expression of a molecule of interest. Advantagously, the original structure of the recipient promoter is therefore not disrupted in a way that might lead to undesired side-effects.

In another embodiment, the promoter activating nucleic acid sequence described above comprises or consist of one or more contiguous stretch(es) of nucleotides isolated from a donor promoter, wherein the donor promoter is a promoter of a gene having a high expression level.

The gene having a high expression level has an expression level comparable to the expression levels of the about 250 most active genes such as the S-adenosyl methionine decarboxylase 2 (SAM2, GRMZM2G154397) in a certain organism. Preferably, the gene has an average FPKM value of >1000 in different tissues or under different genomic and/or environmental conditions.

Each of the one or more contiguous stretch(es) described above may be at least 90%, 91%, 92%, 93%, 94%, 95%, 96%, 97%, 98%, or 99% identical to or may be identical to the core promoter sequence of the donor promoter over the whole length of the one or more contiguous stretch(es).

One contiguous stretch may correspond to the whole core promoter sequence of the donor promoter. However, it may also only represent a shorter section of the core promoter or two or more shorter sections of the core promoter sequences, which are not adjacent in then donor promoter.

In another embodiment, each of the one or more contiguous stretch(es) described above is at least 90%, 91%, 92%, 93%, 94%, 95%, 96%, 97%, 98%, or 99% identical to or is identical to a sequence of the same length from position -50 to position +20 relative to the transcription start site of the donor promoter over the whole length of each stretch.

As demonstrated below, one or more contiguous stretch(es), can be taken from a donor promoter sequence and inserted or introduced into a recipient promoter to activate the latter. One contiguous stretch of nucleotides from the core promoter can also be inserted or introduced two or more times into one recipient promoter resulting in a desired activation.

In one embodiment, each of the one or more contiguous stretch(es) described above comprises at least 6, at least 7, at least 8, at least 9 or at least 10 nucleotides or has a length of six or more nucleotides.

Further preferred are promoter activating nucleic acid sequences which consist of two, three, four, five or more contiguous stretch(es) of 20 or less nucleotides length each, preferably 15 or less nucleotides length each, more preferably 10 or less nucleotides length each, which were isolated from a donor promoter. These stretches may be the same or different and they may be inserted and/or introduced at different positions and in varying order into the recipient promoter.

If only short stretches are used, it is possible to enhance the expression of a nucleic acid molecule of interest by many times with only minimal modification of the recipient promoter. Preferably, the contiguous stretches are 20 nucleotides or shorter, more preferably 10 nucleotides or shorter.

In a further embodiment, the promoter activating nucleic acid sequence described above comprises one or more TATA box motif(s) of the donor promoter or one or more TATA box motif(s) having a relative score of greater than 0.8, greater than 0.81, greater than 0.82, greater than 0.83, greater than 0.84, greater than 0.85, greater than 0.86, greater than 0.87, greater than 0.88, greater than 0.89, or greater than 0.90, preferably greater than 0.91, greater than 0.92, greater than 0.93, greater than 0.94, or greater than 0.95, more preferably greater than 0.96, greater than 0.97, greater than 0.98, or greater than 0.99, when matching or aligning the promoter activating nucleic acid sequence to the TATA box consensus. In a preferred embodiment, one or more TATA box motif(s) of the donor promoter are modified by addition, substitution or deletion of one or more nucleotides for converting the one or more TATA box motif(s) of the donor promoter into one or more TATA box motif(s) having increased or higher relative score(s) when matching or aligning the promoter activating nucleic acid sequence to the TATA box consensus. A TATA box motif may also be present in any or all of the contiguous stretch(es) described above. It was demonstrated in the context of the present invention, that promoter activating sequences with a length of 20 nucleotides or less comprising a TATA box motif have the strongest activation properties. However, also sequences without a TATA box motif showed significant activating properties, a TATA box motif is therefore not strictly required to be present in a promoter activating nucleic acid sequence as described herein.

In another embodiment, the promoter activating nucleic acid sequence described above comprises one or more Y patch promoter element(s) of the donor promoter.

In yet another embodiment, the promoter activating nucleic acid sequence described above comprises one or more TATA box motif(s) of the donor promoter or one or more TATA box motif(s) having a relative score of greater than 0.8, greater than 0.81, greater than 0.82, greater than 0.83, greater than 0.84, greater than 0.85, greater than 0.86, greater than 0.87, greater than 0.88, greater than 0.89, or greater than 0.90, preferably greater than 0.91, greater than 0.92, greater than 0.93, greater than 0.94, or greater than 0.95, more preferably greater than 0.96, greater than 0.97, greater than 0.98, or greater than 0.99, when matching or aligning the promoter activating nucleic acid sequence to the TATA box consensus, and one or more Y patch promoter element(s) of the donor promoter.

In one embodiment, the promoter activating nucleic acid sequence described above has a sequence identity of at least 75%, 80%, 85%, 90%, 95%, 96%, 97%, 98%, or 99% to one of the sequences of SEQ ID NOs: 1 to 30, GTATAAAAG (E59), CTATAAATA (E59a), CTATATATA (E59b), CTATAAAAA (E59c) and CTATATAAA (E59d), preferably over the whole length of the promoter activating nucleic acid sequence, preferably SEQ ID NO: 1, SEQ ID NO: 2, SEQ ID NO: 9, SEQ ID NO: 10, SEQ ID NO: 11, SEQ ID NO: 12, SEQ ID NO: 13, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d), particularly preferably SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d).

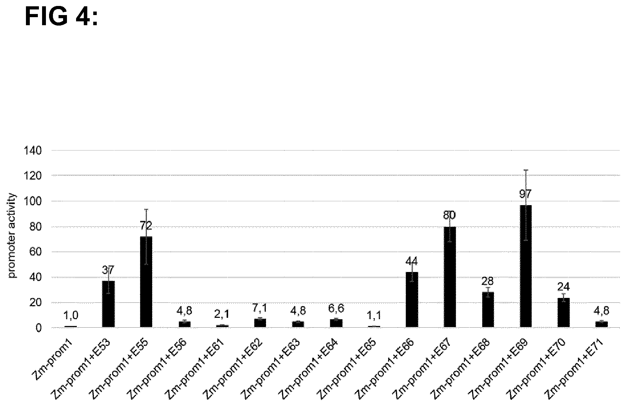

The above listed sequences have been selected as described in the context of the examples below and have been tested for their promoter activating activity. The sequences of SEQ ID NOs 1 to 14 and 20 to 30 represent approximately 60 nucleotide long core promoter sequences shown in Table 1 below. The achieved activation of selected sequences is partly shown in Figure 4.

**Table 1: Sequences of -60 nucleotide long promoter activating nucleic acid sequences isolated from core promoters and tested**

| **name** | **SEQ ID NO** | **Sequence** |
|---|---|---|
| E53 | 1 | AACCCGGACCCGGTAGGAAGGAGCTATAAAGACAAGCCAAACGAGGGCATCCCTTCT |
| E55 | 2 | CGCTATAAAATATCCCCACGCTGCTTCGCCCTGCCCACCACAGCATCCGCAGTTCCC |
| E56 | 3 | TGCTGTTAGCGGTATAAAAAGCGGAAACCCTAGCATTCGCCGCGAGCTTATCACTTA |
| E61 | 4 | GGGACTCGGCGACAGGCCTTTTGTAGACCGCAGCCGGCACCATCTCTTGCCGCACCCCCC |
| E62 | 5 | CCCCTCTTAAAAGCCGCCTCTCGCCGCCGCCCGCAAACCCTCATTTTTCTCTCTCCTGCG |
| E63 | 6 | CCACCATAAATGCGCCGCGGCCGTCCTCGCTGCCCAACCCTTGCTCGCTGCGCCGCCGCC |
| E64 | 7 | GGCGTTAATATCTCCCCTCCCTTCCCTCTTCTGGTCTCCGCCCCGCTCCTTGCCTCCGAT |
| E65 | 8 | CGTTTTTTTTACGCTGTCAATGCATAACCTGCGTTGGCATTCCGCCTGCTGGACTTCCTC |
| E66 | 9 | CGCCCGCCGTCATAAATAGCCAGCCCCATCCCCAGCTTCTTTCCCCAACCTCATCTTCTC |
| E67 | 10 | CGCCCGCCGTAATAAATAGACACCCCCTCCACACCCTCTTTCCCCAACCTCGTGTTGTTC |
| E68 | 11 | TGCTGCTAGCAGTATAAATATGCTGAAAGCCTGAAACCCTAGGCGAAGCTTATCGCTTAT |
| E69 | 12 | GTCGGCTTTAAAAGGACACGAGCGCTTAAACCCCCACCCCATATCCGCATCCGCTGCCTC |
| E70 | 13 | GCCGGCTTTAAAAACGCACACAAGCGCTAAAACCCTCTCCACCGTCCACCTCAGCTCCCA |
| E71 | 14 | CCCGACTACATCAACCAACGCGTATCGGCGGTGGCAAACCCTCTAGCTTCCCACTCCGCT |
| E73 | 20 | ATGTAAAAAAAAAGCTTATATAAAGGGAATCAGACATGAGGTTTTGGCATAAAAACTATC |
| E74 | 21 | CCCCCTCACCCCTACATATACACCACTCTCTCCTTCAATCTTCTTCATCACTCTCATTTT |
| E75 | 22 | GCGTAATTATGAACGTTATATAAACCGGTTACAATTACAACCTATCACACCAAAAAGCAA |
| E76 | 23 | CCACCTCCTTCAAACCTATTTATACTCCCTCACCTCCTTCACTACCTCCTCGCTTCACCC |
| E77 | 24 | TCTACACTTCCTTTAGTATATTTAGCCTCAAATTACTACTGGTCACTTATACATTTCTCA |
| E78 | 25 | GTCGGTCAAACAAGTCTTTAAATACAGCCTATTCCCTTCATTGGTTTCTCATCCTTCATT |
| E79 | 26 | ACCCTAAAACACTCCTTATATAATTCACTCCCTCACATTTCAATTTCCGCCTCCTATACT |
| E80 | 27 | GTTTCTCTCTCTCTCCTTTAAATAAAACCCTAACTTTCTTCACCACTCTCACTCACACTC |
| E81 | 28 | CTTCCTCTCCTCAACATAATAAAGGATAGCAAGTCACACATTCAATCGCCTCTCTCTCCT |
| E82 | 29 | GATACTCCATTTCCATTATTTAAGGAGTGCAAGTGTGGGTGTATGAAAGTAAGGTACCAA |
| E83 | 30 | TTCCTCATTTCTCCAGTATAAGAACCACCACCACCCTTGTTCTCCCACAAACGCAAAATC |

The sequences of SEQ ID NOs 15 to 17 and GTATAAAAG represent shortened elements of sequences shown in Table 1, each of which comprise one TATA box motif and maintained activating property. The sequences are shown in Table 2 below.

**Table 2: Shortened elements as promoter activating nucleic acid sequences**

| **name** | **SEQ ID NO** | **Sequence** | **achieved activation** |
|---|---|---|---|
| E53b | 15 | TATAAAGACAAGCCAAACGA | 12 to 26-fold |
| E55a | 16 | GCTATAAAATATCCCCACGC | 42-fold |
| E56a | 17 | GTATAAAAAGCGGAAACCCT | 26-fold |
| E59 | | GTATAAAAG | 20-fold |

The sequences of SEQ ID NOs 18 and 19 are further optimized sequences derived from E53 shown in Table 1. Table 3 below shows how optimization of E53 achieved better activation properties.

**Table 3: Optimization of element E53**

| **name** | **SEQ ID NO** | **Sequence** | Achieved activation |
|---|---|---|---|
| E53 | 1 | AACCCGGACCCGGTAGGAAGGAGCTATAAAGACAAGCCAAACGAGGGCATCCCTTCT | 37-fold |
| E53b | 15 | TATAAAGACAAGCCAAACGA | 12 to 26-fold |
| E53e | 18 | GCTATAAAGACAAGCCAAAC | 33-fold |
| E53f | 19 | GCTATAAAGA--------------GCATCCCTTC | 41-fold |

The sequences CTATAAATA (E59a), CTATATATA (E59b), CTATAAAAA (E59c) and CTATATAAA (E59d) are further optimized sequences derived from E59 shown in Table 2. Table 4 below shows how optimization of TATA box motif of E59 achieved better activation properties. The TATA-box version E59a represents the perfect TATA box consensus sequences for a monocot TATA-box and the versions E59b-d are slightly modified.

**Table 4: Different versions of the shortened activating element E59 having modified TATA box motifs with higher or increased relative scores**

| **name** | **SEQ ID NO** | **Sequence** | **achieved activation** |
|---|---|---|---|
| E59 | | GTATAAAAG | 12 to 37-fold |
| E59a | | CTATAAATA | 62-fold |
| E59b | | CTATATATA | 44-fold |
| E59c | | CTATAAAAA | 38-fold |
| E59d | | CTATATAAA | 46-fold |

The promoter activating nucleic acid sequences described above can be used to activate any chosen target gene. Moreover, they are applicable in any chosen cell, organism or tissue. In the context of the present disclosure it is preferred, that they activate a promoter in a pant cell or plant, more preferably a crop plant.

In one embodiment, the present invention therefore provides a promoter activating nucleic acid sequence as described above, wherein the cell or organism is a plant cell or plant.

In another embodiment, the recipient promoter and/or the donor promoter is/are a plant promoter.

In a further embodiment, the recipient promoter and the donor promoter as described above are different and/or originate from the same species or from different species.

It is possible to use activating sequences from a donor promoter of one species and introduce them into a recipient donor of another species to provide a strong enhancement. This is in particular possible when using different plant species.

In one embodiment, the plant or plant cell or plant promoter described in any of the embodiments above, originates from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell or plant promoter originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

The nucleic acid molecule of interest is preferably a monogenic or polygenic crop trait encoding gene and may be selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content. Specific preferred examples are ZmZEP1 (SEQ ID NO 31), ZmRCA-beta (SEQ ID NO 32), BvEPSPS (SEQ ID NO 33), and BvFT2 (SEQ ID NO 34) (see also Table 5).

**Table 5: Nucleic acid molecule of interest with potential recipient promoters**

| **name** | **Gene ID** | **SEQ ID NO** |
|---|---|---|
| ZmZEP1 (Zm-prom2) | GRMZM2G127139 | 31 |
| ZmRCA-beta (Zm-prom3) | GRMZM2G162200 | 32 |
| BvEPSPS | g34414 | 33 |
| BvFT2 | g2128 | 34 |

It has been demonstrated as described in further detail below that by inserting or introducing and optionally further modification the promoter activating nucleic acid sequences described above, it is possible to increase the expression level of the nucleic acid molecule of interest by several fold compared to the expression level of the nucleic acid molecule of interest under the control of the recipient promoter without manipulation. This finding provides a significant advantage. Moreover, it had previously not been demonstrated that such promoter activating elements could be transferred to other promoters.

In one embodiment, upon site-specific insertion or introduction and optionally further modification, of the promoter activating nucleic acid sequence(s) described above into the recipient promoter, the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the inserted or introduced promoter activating nucleic acid sequence.

In another aspect, the present invention provides a chimeric promoter comprising a recipient promoter or the core promoter thereof and at least one promoter activating nucleic acid sequence as described in any of the embodiments above inserted or introduced at a position upstream or downstream of the transcription start site of the recipient promoter.

A chimeric promoter according to the present invention comprises an activating nucleic acid sequence that is not natively found in the promoter. The activating nucleic acid sequence may have been inserted or introduced by any means of modification of the recipient promoter sequence such as insertion of one or more contiguous stretch(es) representing the promoter activating nucleic acid sequence or addition of one or more nucleotides to the recipient promoter, or deletion or substitution of one or more nucleotides of the recipient promoter or any combination of the above modifications, which result in the promoter activating nucleic acid sequence being introduced. Five possible options to insert or introduce an activating sequence are shown in Figure 1. The presence of the promoter activating nucleic acid sequence leads to an increased expression level of any gene that is placed under the control of the chimeric promoter with respect to the expression level of the same gene under the control of the promoter without the manipulation. Notably, the increase in expression levels is at least 2-fold but can be up to 40-fold and more.

In one embodiment, a chimeric promoter as described above is provided, wherein the promoter activating nucleic acid sequence is inserted or introduced by addition and/or deletion and/or substitution of one or more nucleotides into the recipient promoter at a position
i. 500 nucleotides or less, preferably 150 nucleotides or less upstream of the transcription start site, and/or
ii. 50 or more nucleotides upstream of the start codon; and/or
iii. where there is no upstream open reading frame (uORF) downstream of the insertion or introduction site.

The above specified positions i. to iii. are to be understood to refer to the unmodified recipient promoter sequence. The transcription start site of position i. represents the transcription start site of the nucleic acid molecule, which is under the control of the recipient promoter and the start codon of position ii. represents the start codon of this nucleic acid molecule.

In the context of the present invention, different introduction sites and insertion sites, have been tested and it has been found out that the specific site does have an influence on the activating effect of the promoter activating sequences and is thus not completely arbitrary. The above rules i. to iii. have been elucidated in these tests to achieve an activating effect.

In another embodiment, the chimeric promoter comprises a recipient promoter or the core promoter thereof and at least one promoter activating nucleic acid sequence as described above inserted or introduced at a position upstream of the transcription start site of the recipient promoter between -91 and -1, between -53 and -1 or between -43 and -1 or downstream of the transcription start site of the recipient promoter between +1 and +91, between +1 and +50 or between +1 and +42.

In a preferred embodiment, the chimeric promoter comprises a recipient promoter or the core promoter thereof and at least one promoter activating nucleic acid sequence as described above inserted or introduced at a position upstream or downstream of the transcription start site of the recipient promoter, wherein the distance between promoter activating nucleic acid sequence and the start codon is at least 70 nucleotides, preferably at least 100 nucleotides, more preferably at least 120 nucleotides.

In a further aspect, the present invention provides a delivery system comprising the promoter activating nucleic acid sequence and/or the chimeric promoter as described in any of the embodiments above, and/or means for site-specific insertion or site-specific introduction of the promoter activating nucleic acid sequence into a recipient promoter.

The promoter activating nucleic acid sequence disclosed herein need to be introduced into the genome of the cell or the organism, in which they are desired to increase the expression of a target gene. The skilled person is aware of a large number of delivery techniques and the corresponding systems to introduce the promoter activating nucleic acid sequence or the chimeric promoter into the genome of a cell or organism in a targeted way so that they can perform the desired function. The promoter activating nucleic acid sequence can be inserted or introduced by addition and/or deletion and/or substitution of sequence stretches or single nucleotides or a combination of any of the above modifications. Tools for site-specific modification of nucleic acid sequences to achieve insertion or introduction of the promoter activating nucleic acid sequence include site-specific nucleases, recombinases, transposases or base editors. If only a very few nucleotides need to be modified TILLING can also be applied to change existing sequences into promoter activating nucleic acid sequences as described above. These tools and the corresponding techniques are described in more detail below.

In yet another aspect, the present invention provides a nucleic acid construct or an expression cassette comprising the promoter activating nucleic acid sequence as described in any of the embodiments above and/or the chimeric promoter as described in any of the embodiments above.

After its import, e.g. by transformation or transfection by biological or physical means, the nucleic acid construct or the expression cassette can either persist extrachromosomally, i.e. non integrated into the genome of the target cell, for example in the form of a double-stranded or single-stranded DNA, a double-stranded or single-stranded RNA. Alternatively, the construct, or parts thereof, according to the present disclosure can be stably integrated into the genome of a target cell, including the nuclear genome or further genetic elements of a target cell, including the genome of plastids like mitochondria or chloroplasts. A nucleic acid construct or an expression cassette may also be integrated into a vector for delivery into the target cell or organism.

In a further aspect, the present invention provides a vector comprising the promoter activating nucleic acid sequence as described in any of the embodiments above and/or the chimeric promoter as described in any of the embodiments above and/or the nucleic acid construct or the expression cassette as described above, and/or means for site-specific introduction of the promoter activating nucleic acid sequence as described above into a recipient promoter.

Besides transformation methods based on biological approaches, like *Agrobacterium* transformation or viral vector mediated plant transformation, methods based on physical delivery methods, like particle bombardment or microinjection, have evolved as prominent techniques for importing genetic material into a plant cell or tissue of interest. Helenius et al. ("Gene delivery into intact plants using the HeliosTM Gene Gun", Plant Molecular Biology Reporter, 2000, 18 (3):287-288) discloses a particle bombardment as physical method for transferring material into a plant cell. Currently, there are a variety of plant transformation methods to introduce genetic material in the form of a genetic construct into a plant cell of interest, comprising biological and physical means known to the skilled person on the field of plant biotechnology and which can be applied. Notably, said delivery methods for transformation and transfection can be applied to introduce the required tools simultaneously. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, e.g., cationic liposomes, or transfection with cationic polymers, including DEAD-dextran or polyethylenimine, or combinations thereof. Every delivery method has to be specifically fine-tuned and optimized so that a construct of interest can be introduced into a specific compartment of a target cell of interest in a fully functional and active way. The above delivery techniques, alone or in combination, can be used to introduce the promoter activating sequences according to the present invention or the constructs, expression cassettes or the vectors carriying the requreid tools i.e. a site-specific effector complex or at least one subcomponent thereof, i.e., at least one site-specific nuclease, at least one guide RNA, at least one repair template, or at least one base editor, or the sequences encoding the aforementioned subcomponents, according to the present invention into a target cell, *in vivo* or *in vitro.*

In another aspect, the present invention provides a cell or organism or a progeny thereof or a part of the organism or progeny thereof,
a) in which a promoter activating nucleic acid as described in any of the embodiments above is inserted or introduced by addition and/or deletion and/or substitution of one or more nucleotides into a recipient promoter controlling the expression of a nucleic acid molecule of interest in the cell or the organism, preferably inserted or introduced at a position upstream or downstream of the transcription start site of the recipient promoter, more preferably introduced at a position
   i. 500 nucleotides or less, preferably 150 nucleotides or less upstream of the transcription start site of the nucleic acid molecule of interest, and/or
   ii. 50 or more nucleotides upstream of the start codon of the nucleic acid molecule of interest; and/or
   iii. where there is no upstream open reading frame (uORF) downstream of the insertion or introduction site; or
b) comprising the chimeric promoter as described in any of the embodiments above, the delivery system as described in any of the embodiments above, the nucleic acid construct or an expression cassette as described above and/or the vector as described above.

In one embodiment of the cell or organism or the progeny thereof or the part of the organism or progeny thereof, the promoter activating nucleic acid is inserted or introduced at a position upstream of the transcription start site of the recipient promoter between -91 and -1, between -53 and -1 or between -43 and -1 or downstream of the transcription start site of the recipient promoter between +1 and +91, between +1 and +50 or between +1 and +42.

In a preferred embodiment of the cell or organism or the progeny thereof or the part of the organism or progeny thereof, the promoter activating nucleic acid sequence is inserted or introduced at a position upstream or downstream of the transcription start site of the recipient promoter, wherein the distance between promoter activating nucleic acid sequence and the start codon of the nucleic acid molecule of interest is at least 70 nucleotides, preferably at least 100 nucleotides, more preferably at least 120 nucleotides.

The cell or organism described above is capable of expressing the molecule of interest in an amount that is several fold that of the expression achieved with the unmanipulated recipient promoter. Thus, it is possible to e.g. significantly improve certain traits in the organism. In the context of the present invention, it is particuarly preferred that the cell or organism is a plant cell or a plant.

In one embodiment, in the cell or organism or the progeny thereof or the part of the organism or the progeny thererof as described above, the recipient promoter is a plant promoter.

Advantageously, it is possible by using the promoter activating nucleic acid sequence to increase the expression of endogenous or exogenous nucleic acid molecules of interest, which may be either under the control of their native or a heterologous promoter. Thus, endogenous traits can be specifically enhanced or exogenous traits can be introduced and expressed at high levels.

In another embodiment, in the cell or organism or the progeny thereof or the part of the organism or the progeny thererof as described above, the nucleic acid molecule of interest is therefore heterologous or native to the recipient promoter and/or is an endogenous or exogenous nucleic acid molecule to the cell or organism.

In the context of the present invention, it is particularly preferred to increase the expression of the nucleic acid molecule of interest in a plant cell or plant, particularly a crop plant.

In a further embodiment, the cell or organism or the progeny thereof or the part of the organism or the progeny thererof according to any of the embodiments described above, is a plant cell or plant or part thereof, preferably wherein the plant originates from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In a further aspect, the present invention provides a method for identifying a promoter activating nucleic acid sequence or a chimeric promoter as described in any of the embodiments above. Thus, the present invention allows a skilled person to identify promoter activating nucleic acid sequences, which can be used to increase the expression levels of a gene of interest.

The present invention therefore relates to a method for identifying a promoter activating nucleic acid sequence or a chimeric promoter, preferably a promoter activating nucleic acid sequence or a chimeric promoter as described in any of the emodiments above, comprising:
i) identifying a gene in a cell or an organism having a high expression level,
ii) isolating one or more contiguous stretch(es) from the promoter of the gene identified in step i) wherein the one or more contiguous stretch(es) originate(s) a) from the core promoter of the said donor promoter or b) from a sequence from position -50 to position +20 relative to the transcription start site of said donor promoter,
iii) inserting or introducing by addition and/or deletion and/or substitution of one or more nucleotides the one or more contiguous stretch(es) into a recipient promoter controlling the expression of a nucleic acid molecule of interest at a position upstream or downstream of the transcription start site of the recipient promoter,
iv) determining in a cell or organism or *in vitro* the expression level of the nucleic acid molecule of interest under the control of the recipient promoter comprising the insertion or introduction of step iii) relative to the expression level of the same or another nucleic acid molecule of interest under the control of the recipient promoter without the insertion or introduction of step iii) or to another reference promoter in a given environment and/or under given genomic and/or environmental conditions, wherein the nucleic acid molecule of interest is heterologous or native to the recipient promoter and/or is endogenous or exogenous to the cell or organism, and
v) identifying and thus providing the promoter activating nucleic acid sequence as described in any of the embodiments above or the chimeric promoter as described in any of the embodiments above when increased expression of the nucleic acid molecule of interest in step iv) is observed,
vi) optionally, shortening the promoter activating nucleic acid sequence identified in step v) stepwise and repeating steps iv) and v) at least one time and/or modifying one or more TATA box motif(s) present in the promoter activating nucleic acid sequence identified in step v) or in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus, and repeating steps iv) and v) at least one time.

The gene identified in step i) has an expression level comparable to the expression levels of the about 250 most active genes such as the S-adenosyl methionine decarboxylase 2 (SAM2, GRMZM2G154397). Preferably, the gene has an average FPKM value of >1000 in different tissues or under different genomic and/or environmental conditions.

Each of the one or more contiguous stretch(es) described above may be identical to or at least 90%, 91%, 92%, 93%, 94%, 95%, 96%, 97%, 98%, or 99% identical to the core promoter sequence of the donor promoter over the whole length of the one or more contiguous stretch(es).

In another embodiment, each of the one or more contiguous stretch(es) described above is at least 90%, 91%, 92%, 93%, 94%, 95%, 96%, 97%, 98%, or 99% identical to or is identical to a sequence of the same length from position -50 to position +20 relative to the transcription start site of the donor promoter over the whole length of each stretch.

After site-specific insertion or introduction into the recipient promoter, the one or more contiguous stretch(es) are tested for their promoter activating properties. The testing can be done *in vivo* or *in vitro* comparing the expression level of a nucleic acid molecule of interest, e.g. a reporter gene, under the control of the recipient promoter comprising the insertion or the introduction with the expression level of the same or another nucleic acid molecule of interest under the control of the recipient promoter without the insertion or the introduction or to another reference promoter in a given environment and/or under given genomic and/or environmental conditions, so that a difference in the expression levels can be determined. The recipient promoter may be the promoter that natively controls the expression of the nucleic acid molecule of interest or the nucleic acid molecule of interest may be placed under the control of a heterologous recipient promoter, which does not natively control its expression. If the testing is performed *in vivo,* the nucleic acid molecule of interest may be endogenous to the cell or organism that it is expressed in. In this case the recipient promoter may be the promoter that natively controls the expression of this nucleic acid molecule of interest in the cell or organism but it is also possible that the endogenous nucleic acid molecule of interest is under the control of a heterologous recipient promoter, which does not natively control its expression. Alternatively, the nucleic acid molecule of interest may be exogenous to the cell or organism that it is tested in. In this case the recipient promoter may also be exogenous to the cell or organism but it may be the promoter that the nucleic acid molecule of interest is controlled by in its native cellular environment. On the other hand, the recipient promoter may also be exogenous to the cell or organism in that it tested and at the same time be hererologous to the nucleic acid molecule of interest.

If an increased expression level of the nucleic acid molecule of interest under the control of the promoter having the insertion or introduction is observed in step iv), the contigous stretch(es) is/are identified as promoter activating nucleic acid sequences as described above or the recipient promoter carrying the contiguous stretch(es) is identified as a chimeric promoter as described above.

The promoter activating nucleic acid sequences identified in step v) may optionally be shortened or optimized by stepwise removal of nucleotides from the ends or from within the sequence, inserting or introducing them in the recipient promoter and testing for a loss or a gain of promoter activating properties by repeating steps iv) and v). Thus, very short but highly efficient promoter activating nucleic acid sequences can be provided, which can be introduced by minimal modification of the recipient promoter. The shortened sequences or contiguous stretch(es) preferably have a length between 6 and 40 nucleotides, more preferably between 9 and 20 nucleotides.

The promoter activating nucleic acid sequences identified in step v) may optionally be optimized by modification of one or more TATA box motif(s) present in the promoter activating nucleic acid sequence identified in step v) or in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus.

In one embodiment of the method described above, in step iii) the one or more contiguous stretch(es) is/are inserted or introduced into the recipient promoter at a position
(a) 500 nucleotides or less, preferably 150 nucleotides or less upstream of the transcription start site of the the nucleic acid molecule of interest; and/or
(b) more than 50 nucleotides upstream of the start codon of the the nucleic acid molecule of interest; and/or
(c) where there is no upstream open reading frame (uORF) downstream of the insertion or introduction site.

In a further embodiment of the method described above, the one or more contiguous stretch(es) is/are inserted or introduced at a position upstream of the transcription start site of the recipient promoter between -91 and -1, between -53 and -1 or between -43 and -1 or downstream of the transcription start site of the recipient promoter between +1 and +91, between +1 and +50 or between +1 and +42.

In a preferred embodiment of the method described above, the one or more contiguous stretch(es) is/are inserted or introduced at a position upstream or downstream of the transcription start site of the recipient promoter, wherein the distance between the one or more contiguous stretch(es) and the start codon of the nucleic acid molecule of interest is at least 70 nucleotides, preferably at least 100 nucleotides, more preferably at least 120 nucleotides.

Insertion or introduction of the one or more contiguous stretch(es) isolated in step ii) at a position according to (a), (b) and/or (c) into the recipient promoter is most likely to lead to successful activation as explained above.

In another aspect, the present invention provides a method for increasing the expression level of a nucleic acid molecule of interest in a cell, comprising:
ia) introducing into the cell the promoter activating nucleic acid sequence according to any of the embodiments above, the chimeric promoter as described above, the delivery system as described above, or the nucleic acid construct or an expression cassette as described above, or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any of the embodiments above into a recipient promoter controlling the expression of the nucleic acid molecule of interest in the cell at a position upstream or downstream of the transcription start site of the recipient promoter controlling the expression of the nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of the nucleic acid molecule of interest by addition and/or deletion and/or substitution so that a promoter activating nucleic acid sequence as defined in any of the embodiments above is formed, and
iiic) optionally, modifying one or more TATA box motif(s) present in the promoter activating nucleic acid sequence inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus.

The modification in step iiib) may comprise any modification of the recipient promoter sequence by addition of one or more single nucleotide(s) or a sequence of nucleotides to the sequence of the recipient promoter, or deletion or substitution of one or more single nucleotide or a sequence of nucleotides of the recipient promoter sequence.

The modification in step iiic) may comprise any modification of the recipient promoter sequence or the promoter activating nucleic acid sequence by addition of one or more single nucleotide(s) or a sequence of nucleotides to the sequence of the recipient promoter or to the promoter activating nucleic acid sequence, or deletion or substitution of one or more single nucleotide or a sequence of nucleotides of the recipient promoter sequence or of the promoter activating nucleic acid sequence.

The introduction of step i) may e.g. be achieved by means of transformation, transfection or transduction by biological means, including *Agrobacterium* transformation, or physical means, including particle bombardment as explained in more detail above.

The targeted insertion or introduction or modification of the promoter activating nucleic acid sequence into the recipient promoter may be achieved by aditionally introducing a site-specific nuclease or an active fragment thereof. Site-specific DNA cleaving activities of meganucleases, zinc finger nucleases (ZFNs), transcription activator-like effector nucleases (TALENs), or the clustered regularly interspaced short palindromic repeat (CRISPR), mainly the CRISPR/Cas9 technology have been widely applied site-directed modifications of animal and plant genomes. The nucleases cause double strand breaks (DSBs) at specific cleaving sites, which are repaired by nonhomologous end-joining (NHEJ) or homologous recombination (HR), which allows e.g. the introduction of insertions at the cleaving site. More recently discovered CRISPR systems include CRISPR/Cpf1, CRISPR/C2c2, CRISPR/CasX, CRISPR/CasY and CRISPR/Cmr. Recombinases and Transposases catalyze the exchange or relocation of specific target sequences and can therefore also be used to create targeted modifications.

CRISPRs (Clustered Regularly Interspaced Short Palindromic Repeats) in their natural environment originally evolved in bacteria where the CRISPR system fulfils the role of an adaptive immune system to defend against viral attack. Upon exposure to a virus, short segments of viral DNA are integrated into the CRISPR locus. RNA is transcribed from a portion of the CRISPR locus that includes the viral sequence. That RNA, which contains sequence complementary to the viral genome, mediates targeting of a CRISPR effector protein to a target sequence in the viral genome. The CRISPR effector protein cleaves and thereby interferes with replication of the viral target. Over the last years, the CRISPR system has successfully been adapted for gene editing or genome engineering also in eukaryotic cells.

A CRISPR system in its natural environment describes a molecular complex comprising at least one small and individual non-coding RNA in combination with a Cas nuclease or another CRISPR nuclease like a Cpf1 nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into 2 classes comprising five types of CRISPR systems, the type II system, for instance, using Cas9 as effector and the type V system using Cpf1 as effector molecule (Makarova et al., Nature Rev. Microbial., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova et al., 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which systems contain both a crRNA and also a trans-activating RNA (tracrRNA) as the guide motif. These hybridize and form double-stranded (ds) RNA regions which are recognized by RNAse III and can be cleaved in order to form mature crRNAs. These then in turn associate with the Cas molecule in order to direct the nuclease specifically to the target nucleic acid region. Recombinant gRNA molecules can comprise both, the variable DNA recognition region and also the Cas interaction region, and can be specifically designed, independently of the specific target nucleic acid and the desired Cas nuclease. As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al., "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpf1 nucleases it has been described that the Cpf1-crRNA complex efficiently cleaves target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized.

Presently, for example, Type II systems relying on Cas9, or a variant or any chimeric form thereof, as endonuclease have been modified for genome engineering. Synthetic CRISPR systems consisting of two components, a guide RNA (gRNA) also called single guide RNA (sgRNA) and a non-specific CRISPR-associated endonuclease can be used to generate knock-out cells or animals by co- expressing a gRNA specific to the gene to be targeted and capable of association with the endonuclease Cas9. Notably, the gRNA is an artificial molecule comprising one domain interacting with the Cas or any other CRISPR effector protein or a variant or catalytically active fragment thereof and another domain interacting with the target nucleic acid of interest and thus representing a synthetic fusion of crRNA and tracrRNA ("single guide RNA" (sgRNA) or simply "gRNA"; Jinek et al., 2012, supra). The genomic target can be any -20 nucleotide DNA sequence, provided that the target is present immediately upstream of a PAM. The PAM sequence is of outstanding importance for target binding and the exact sequence is dependent upon the species of Cas9 and, for example, reads 5' NGG 3' or 5' NAG 3' (Standard IUPAC nucleotide code) (Jinek et al., 2012, supra) for a *Streptococcus pyogenes* derived Cas9. Using modified Cas nucleases, targeted single strand breaks can be introduced into a target sequence of interest.

Once expressed, the Cas9 protein and the gRNA form a ribonucleoprotein complex through interactions between the gRNA "scaffold" domain and surface-exposed positively-charged grooves on Cas9. Importantly, the "spacer" sequence of the gRNA remains free to interact with target DNA. The Cas9-gRNA complex will bind any genomic sequence with a PAM, but the extent to which the gRNA spacer matches the target DNA determines whether Cas9 will cut. Once the Cas9-gRNA complex binds a putative DNA target, a "seed" sequence at the 3' end of the gRNA targeting sequence begins to anneal to the target DNA. If the seed and target DNA sequences match, the gRNA will continue to anneal to the target DNA in a 3' to 5' direction (relative to the polarity of the gRNA).

Recently, engineered CRISPR/Cpf1 systems in addition to CRISPR/Cas9 systems become more and more important for targeted genome engineering (see Zetsche et al., supra and EP 3 009 511 A2). The Type V system together with the Type II system belongs to the Class 2 CRISPR systems (Makarova and Koonin Methods. Mol. Biol., 2015, 1311:47-753). The Cpf1 effector protein is a large protein (about 1,300 amino acids) that contains a RuvC like nuclease domain homologous to the corresponding domain of Cas9 along with a counterpart to the characteristic arginine-rich cluster of Cas9. However, Cpf1 lacks the HNH nuclease domain that is present in all Cas9 proteins, and the RuvC-like domain is contiguous in the Cpf1 sequence, in contrast to Cas9 where it contains long inserts including the HNH domain (Chylinski et al. (2014). Classification and evolution of type II CRISPR-Cas systems. Nucleic acids research, 42(10), 6091-6105.; Makarova, 2015). Cpf1 effectors possess certain differences over Cas9 effectors, namely no requirement of additional trans-activating crRNAs (tracrRNA) for CRISPR array processing, efficient cleavage of target DNA by short T-rich PAMs (in contrast to Cas9, where the PAM is followed by a G-rich sequence), and the introduction of staggered DNA double strand breaks by Cpf1. Very recently, additional novel CRISPR-Cas systems based on CasX and CasY have been identified which due to the relatively small size of the effector protein are of specific interest for many gene editing or genome engineering approaches (Burstein et al., "New CRISPR-Cas systems from uncultivated microbes", Nature, December 2016).

Furthermore, a base editing technique can be used to introduce the promoter activating nucleic acid into the recipient promoter. As shown in Figure 1 B1, B2, C, E1 and E2, it is possible to activate a recipient promoter by editing only a few nucleotides. Any base editor or site-specific effector, or a catalytically active fragment thereof, or any component of a base editor complex or of a site-specific effector complex as disclosed herein can be introduced into a cell as a nucleic acid fragment, the nucleic acid fragment representing or encoding a DNA, RNA or protein effector, or it can be introduced as DNA, RNA and/or protein, or any combination thereof.

A key toolset that eliminates the requirement for making selectable modifications with an endonuclease, a DSB, and a repair template is the use of base editors or targeted mutagenesis domains. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al ((2017). Programmable base editing of A• T to G• C in genomic DNA without DNA cleavage. Nature, 551(7681), 464.) described adenine base editors (ABEs) that mediate the conversion of A•T to G•C in genomic DNA.

Any base editing complex according to the present invention can thus comprise at least one cytidine deaminase, or a catalytically active fragment thereof. The at least one base editing complex can comprise the cytidine deaminase, or a domain thereof in the form of a catalytically active fragment, as base editor.

In another embodiment, the at least one first targeted base modification is a conversion of any nucleotide C, A, T, or G, to any other nucleotide. Any one of a C, A, T or G nucleotide can be exchanged in a site-directed way as mediated by a base editor, or a catalytically active fragment thereof, to another nucleotide. The at least one base editing complex can thus comprise any base editor, or a base editor domain or catalytically active fragment thereof, which can convert a nucleotide of interest into any other nucleotide of interest in a targeted way.

The nucleic acid molecule of interest may be endogenous to the cell or organism that it is expressed in. In this case the recipient promter may be the promoter that natively controls the expression of this nucleic acid molecule of interest in the cell or organism but it is also possible that the endogenous nucleic acid molecule of interest is under the control of a heterologous promoter, which does not natively control its expression. Alternatively, the nucleic acid molecule of interest is exogenous to the cell or organism that it is expressed in. In this case the promoter may also be exogenous to the cell or organism but it may be the promoter that the nucleic acid molecule of interest is controlled by in its native cellular environment. On the other hand, the promoter may also be exogenous to the cell or organism in that it is to be activated and at the same time be hererologous to the nucleic acid molecule of interest.

In the method described above, the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the inserted or introduced promoter activating nucleic acid sequence.

The insertion or modification to introduce the promoter activating nucleic acid sequence into the recipient promoter in step iiia) or iiib) of the method is preferably at a position
(a) 500 nucleotides or less, preferably 150 nucleotides or less upstream of the transcription start site of the nucleic acid molecule of interest; and/or
(b) more than 50nucleotides upstream of the start codon of the nucleic acid molecule of interest; and/or
(c) where there is no upstream open reading frame (uORF) downstream of the insertion or introduction site.

The postions (a), (b) and (c) are given with reference to the recipient promoter without the insert or modification.

In a further embodiment of the method for increasing the expression level as described above, the promoter activating nucleic acid sequence is inserted or introduced at a position upstream of the transcription start site of the recipient promoter between -91 and -1, between -53 and -1 or between -43 and -1 or downstream of the transcription start site of the recipient promoter between +1 and +91, between +1 and +50 or between +1 and +42.

In a preferred embodiment of the method for increasing the expression level as described above, the promoter activating nucleic acid sequence is inserted or introduced at a position upstream or downstream of the transcription start site of the recipient promoter, wherein the distance between the one or more contiguous stretch(es) and the start codon of the nucleic acid molecule of interest is at least 70 nucleotides, preferably at least 100 nucleotides, more preferably at least 120 nucleotides.

In one embodiment, the promoter activating nucleic acid sequence has a length between 6 and 70 nucleotides, preferably between 7 and 60 nucleotides, more preferably between 8 and 40 nucleotides and most preferably between 9 and 20 nucleotides.

In one embodiment, the promoter activating nucleic acid sequence inserted or introduced by modification in step iiia) or iiib) as described in any of the embodiments above comprises or consist of one or more contiguous stretch(es) of nucleotides isolated from a donor promoter, wherein the donor promoter is a promoter of a gene having a high expression level.

The gene having a high expression level has an expression level comparable to the expression levels of the about 250 most active genes such as the S-adenosyl methionine decarboxylase 2 (SAM2, GRMZM2G154397). Preferably, the gene has an average FPKM value of >1000 in different tissues or under different genomic and/or environmental conditions.

In a further embodiment, each of the one or more contiguous stretch(es) described above is identical to or at least 90%, 91%, 92%, 93%, 94%, 95%, 96%, 97%, 98%, or 99% identical to the core promoter sequence of the donor promoter over the whole length of the one or more contiguous stretch(es).

In yet another embodiment, each of the one or more contiguous stretch(es) described in any of the embodiments above is identical or at least 90%, 91%, 92%, 93%, 94%, 95%, 96%, 97%, 98%, or 99% identical to a sequence of the same length from position -50 to position +20 relative to the transcription start site of the donor promoter over the whole length of each stretch.

In another embodiment, each of the one or more contiguous stretch(es) described in any of the embodiments above comprises at least 6, at least 7, at least 8, at least 9 or at least 10 nucleotides, or has a length of six or more nucleotides.

In a further embodiment, the promoter activating nucleic acid sequence inserted or introduced as described in any of the embodiments above in step iiia) or iiib) comprises one or more TATA box motif(s) of the donor promoter.

In yet another embodiment, the promoter activating nucleic acid sequence inserted or introduced as described in any of the embodiments above in step iiia) or iiib) has a sequence identity of at least 75%, 80%, 85%, 90%, 95%, 96%, 97%, 98%, or 99% to one of the sequences of SEQ ID NOs: 1 to 30, GTATAAAAG (E59), CTATAAATA (E59a), CTATATATA (E59b), CTATAAAAA (E59c) and CTATATAAA (E59d), preferably over the whole length of the promoter activating nucleic acid sequence, preferably SEQ ID NO: 1, SEQ ID NO: 2, SEQ ID NO: 9, SEQ ID NO: 10, SEQ ID NO: 11, SEQ ID NO: 12, SEQ ID NO: 13, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d), particularly preferably SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d).

In another embodiment of the method described above, the recipient promoter and/or the donor promoter is/are a plant promoter.

In a further embodiment, the recipient promoter and the donor promoter are different and/or originate from the same species or from different species.

In one embodiment, the plant or plant cell or plant promoter described in any of the embodiments of the method described above, originates from a genus selected from the group consisting of *Hordeum*, *Sorghum*, *Saccharum*, Zea, *Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya*, *Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia*, *Allium*, *or Helianthus*, preferably, the plant or plant cell or plant promoter originates from a species selected from the group consisting of *Hordeum vulgare, Hordeum bulbusom*, *Sorghum bicolor*, *Saccharum officinarium*, Zea *spp*., *including* Zea *mays*, *Setaria italica*, *Oryza minuta*, *Oryza sativa*, *Oryza australiensis*, *Oryza alta, Triticum aestivum, Triticum durum, Secale cereale,* Triticale, *Malus domestica, Brachypodium distachyon, Hordeum marinum, Aegilops tauschii, Daucus glochidiatus*, *Beta spp*., including *Beta vulgaris*, *Daucus pusillus*, *Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In a further aspect, the present invention provides a method for producing a cell or organism having an increased expression level of a nucleic acid molecule of interest, comprising:
ia) introducing into the cell the promoter activating nucleic acid sequence as described in any of the embodiments above, the chimeric promoter as described above, the delivery system as described above, or the nucleic acid construct or an expression cassette as described above, or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any of the embodiments above or the chimeric promoter as defined above into a recipient promoter controlling the expression of the nucleic acid molecule of interest in the cell at a position upstream or downstream of the transcription start site of the recipient promoter controlling the expression of the nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of the nucleic acid molecule of interest by addition and/or deletion and/or substitution so that a promoter activating nucleic acid sequence as defined in any of the embodiments above is formed, and
iiic) optionally, modifying one or more TATA box motif(s) present in the promoter activating nucleic acid sequence or the chimeric promoter inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus, and
iv) obtaining a cell or organism having increased expression level of a nucleic acid molecule of interest upon insertion of the promoter activating nucleic acid sequence as described in any of the embodiments above or upon modification to form the promoter activating nucleic acid sequence as described in any of the embodiments above.

The introduction of step i) may e.g. be achieved by means of transformation, transfection or transduction by biological means, including *Agrobacterium* transformation, or physical means, including particle bombardment as described in more detail above.

The nucleic acid molecule of interest may be endogenous to the cell or organism that it is expressed in. In this case the recipient promter may be the promoter that natively controls the expression of this nucleic acid molecule of interest in the cell or organism but it is also possible that the endogenous nucleic acid molecule of interest is under the control of a heterologous promoter, which does not natively control its expression. Alternatively, the nucleic acid molecule of interest is exogenous to the cell or organism that it is expressed in. In this case the promoter may also be exogenous to the cell or organism but it may be the promoter that the nucleic acid molecule of interest is controlled by in its native cellular environment. On the other hand, the promoter may also be exogenous to the cell or organism in that it is to be activated and at the same time be hererologous to the nucleic acid molecule of interest.

In the method described above, the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the inserted or introduced promoter activating nucleic acid sequence.

The insertion or introduction of the promoter activating nucleic acid sequence into the recipient promoter in step iiia) or iiib) of the method is preferably at a position
(a) 500 nucleotides or less, preferably 150 nucleotides or less upstream of the transcription start site of the nuclecic acid molecule of interest; and/or
(b) more than 50nucleotides upstream of the start codon of the nucleic molecule of interest; and/or
(c) where there is no upstream open reading frame (uORF) downstream of the introduction or insertion site.

In a further embodiment of the method for producing a cell or organism having an increased expression level of a nucleic acid molecule of interest as described above, the promoter activating nucleic acid sequence is inserted or introduced at a position upstream of the transcription start site of the recipient promoter between -91 and -1, between -53 and -1 or between -43 and -1 or downstream of the transcription start site of the recipient promoter between +1 and +91, between +1 and +50 or between +1 and +42.

In a preferred embodiment of the method for producing a cell or organism having an increased expression level of a nucleic acid molecule of interest as described above, the promoter activating nucleic acid sequence is inserted or introduced at a position upstream or downstream of the transcription start site of the recipient promoter, wherein the distance between the one or more contiguous stretch(es) and the start codon of the nucleic acid molecule of interest is at least 70 nucleotides, preferably at least 100 nucleotides, more preferably at least 120 nucleotides.

In one embodiment, the promoter activating nucleic acid sequence has a length between 6 and 70 nucleotides, preferably between 7 and 60 nucleotides, more preferably between 8 and 40 nucleotides and most preferably between 9 and 20 nucleotides.

In one embodiment, the promoter activating nucleic acid sequence inserted or introduced as described in any of the embodiments above in step iiia) or iiib) comprises or consist of one or more contiguous stretch(es) of nucleotides isolated from a donor promoter, wherein the donor promoter is a promoter of a gene having a high expression level.

The gene having a high expression level has an expression level comparable to the expression levels of the about 250 most active genes such as the S-adenosyl methionine decarboxylase 2 (SAM2, GRMZM2G154397). Preferably, the gene has an average FPKM value of >1000 in different tissues or under different genomic and/or environmental conditions.

In a further embodiment, each of the one or more contiguous stretch(es) described above is identical to or at least 90%, 91%, 92%, 93%, 94%, 95%, 96%, 97%, 98%, or 99% identical to the core promoter sequence of the donor promoter over the whole length of the core promoter.

In yet another embodiment, each of the one or more contiguous stretch(es) described in any of the embodiments above is identical to or at least 90%, 91%, 92%, 93%, 94%, 95%, 96%, 97%, 98%, or 99% identical to a sequence of the same length from position -50 to position +20 relative to the transcription start site of the donor promoter over the whole length of each stretch.

In another embodiment, each of the one or more contiguous stretch(es) described in any of the embodiments above comprises at least 6, at least 7, at least 8, at least 9 or at least 10 nucleotides, or has a length of six or more nucleotides. In a further embodiment, the promoter activating nucleic acid sequence inserted ot introduced as described in any of the embodiments above in any of the embodiments above in step iiia) or iiib) comprises one or more TATA box motif(s) of the donor promoter.

In yet another embodiment, the promoter activating nucleic acid sequence inserted or introduced in step iiia) or iiib) in any of the embodiments above has a sequence identity of at least 75%, 80%, 85%, 90%, 95%, 96%, 97%, 98%, or 99% to one of the sequences of SEQ ID NOs: 1 to 30, GTATAAAAG (E59), CTATAAATA (E59a), CTATATATA (E59b), CTATAAAAA (E59c) and CTATATAAA (E59d), preferably over the whole length of the promoter activating nucleic acid sequence, preferably SEQ ID NO: 1, SEQ ID NO: 2, SEQ ID NO: 9, SEQ ID NO: 10, SEQ ID NO: 11, SEQ ID NO: 12, SEQ ID NO: 13, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d), particularly preferably SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d).

In another embodiment of the methods described above, the recipient promoter and/or the donor promoter is/are a plant promoter.

In a further embodiment, the recipient promoter and the donor promoter are different and/or originate from the same species or from different species.

In one embodiment, the plant or plant cell or plant promoter described in any of the embodiments of the methods described above, originates from a genus selected from the group consisting of *Hordeum, Sorghum, Saccharum,* Zea, *Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell or plant promoter originates from a species selected from the group consisting of *Hordeum vulgare, Hordeum bulbusom, Sorghum bicolor, Saccharum officinarium,* Zea *spp*., *including* Zea *mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In another aspect, the present invention provides a method for producing a transgenic cell or transgenic organism having increased expression level of a nucleic acid molecule of interest, comprising:
i) transforming or transfecting a cell with the promoter activating nucleic acid sequence as described in any of the embodiments above, the chimeric promoter as described above, the delivery system as described above, or the nucleic acid construct or an expression cassette as described above, or the vector as descried above; and
ii) optionally, regenerating a transgenic organism from the transgenic cell or a transgenic progeny thereof, and
iii) obtaining a transgenic cell or transgenic organism having increased expression level of a nucleic acid molecule of interest.

In one embodiment of the method described above, the cell or organism is a plant cell or plant or a progeny thereof, preferably wherein the plant originates from a genus selected from the group consisting of *Hordeum, Sorghum, Saccharum,* Zea, *Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell originates from a species selected from the group consisting of *Hordeum vulgare, Hordeum bulbusom, Sorghum bicolor, Saccharum officinarium,* Zea *spp., including* Zea *mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In any of the methods described above, the nucleic acid molecule of interest is preferably a crop trait gene, which may be selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content. Specific preferred examples are ZmZEP1 (SEQ ID NO: 31), ZmRCA-beta (SEQ ID NO: 32), BvEPSPS (SEQ ID NO: 33) and BvFT2 (SEQ ID NO: 34).

In yet another aspect, the present invention provides a cell or organism or a progeny thereof, preferably a plant cell or plant or progeny thereof, obtainable by any of the methods as described above.

In one aspect, the present invention also relates to the use of the promoter activating nucleic acid sequence as described in any of the embodiments above, the chimeric promoter as described above, the delivery system as described above, the nucleic acid construct or the expression cassette as described above or the vector described above for increasing the expression level of a nucleic acid molecule of interest in a cell or organism upon site-specific insertion or introduction into a recipient promoter controlling the expression of the nucleic acid molecule of interest.

In one embodiment, the promoter activating nucleic acid sequence has a length between 6 and 70 nucleotides, preferably between 7 and 60 nucleotides, more preferably between 8 and 40 nucleotides and most preferably between 9 and 20 nucleotides.

In another embodiment, the promoter activating nucleic acid sequence described above comprises or consist of one or more contiguous stretch(es) of nucleotides isolated from a donor promoter, wherein the donor promoter is a promoter of a gene having a high expression level.

The gene having a high expression level has an expression level comparable to the expression levels of the about 250 most active genes such as the S-adenosyl methionine decarboxylase 2 (SAM2, GRMZM2G154397). Preferably, the gene has an average FPKM value of >1000 in different tissues or under different genomic and/or environmental conditions.

In a further embodiment, each of the one or more contiguous stretch(es) described above is identical to or is at least 90%, 91%, 92%, 93%, 94%, 95%, 96%, 97%, 98%, or 99% identical to the core promoter sequence of the donor promoter over the whole length of the core promoter

In yet another embodiment, each of the one or more contiguous stretch(es) described in any of the embodiments above is identical to or is at least 90%, 91%, 92%, 93%, 94%, 95%, 96%, 97%, 98%, or 99% identical to a sequence of the same length from position -50 to position +20 relative to the transcription start site of the donor promoter over the whole length of each stretch.

In another embodiment, each of the one or more contiguous stretch(es) described in any of the embodiments above comprises at least 6, at least 7, at least 8, at least 9 or at least 10 nucleotides, or has a length of six or more nucleotides.

In a further embodiment, the promoter activating nucleic acid sequence described in any of the embodiments above comprises one or more TATA box motif(s) of the donor promoter.

In yet another embodiment, the promoter activating nucleic acid sequence described in any of the embodiments above has a sequence identity of at least 75%, 80%, 85%, 90%, 95%, 96%, 97%, 98%, or 99% to one of the sequences of SEQ ID NOs: 1 to 30, GTATAAAAG (E59), CTATAAATA (E59a), CTATATATA (E59b), CTATAAAAA (E59c) and CTATATAAA (E59d), preferably over the whole length of the promoter activating nucleic acid sequence, preferably SEQ ID NO: 1, SEQ ID NO: 2, SEQ ID NO: 9, SEQ ID NO: 10, SEQ ID NO: 11, SEQ ID NO: 12, SEQ ID NO: 13, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d), particularly preferably SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, GTATAAAAG (E59), CTATAAATA (E59a), CTATATATA (E59b), CTATAAAAA (E59c) and CTATATAAA (E59d).

In one embodiment, the promoter activating nucleic acid sequence as described in any of the embodiments above is used for increasing the expression level of a nucleic acid molecule of interest in a cell or organism upon site-specific insertion or introduction, wherein the cell or organism is a plant cell or plant.

In another embodiment, the recipient promoter and/or the donor promoter is/are a plant promoter.

In a further embodiment, the recipient promoter and the donor promoter are different and/or originate from the same species or from different species.

In one embodiment of the use described above, the plant or plant cell or plant promoter described in any of the embodiments above, originates from a genus selected from the group consisting of *Hordeum, Sorghum, Saccharum,* Zea, *Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,* preferably, the plant or plant cell or plant promoter originates from a species selected from the group consisting of *Hordeum vulgare, Hordeum bulbusom, Sorghum bicolor, Saccharum officinarium,* Zea *spp*., *including* Zea *mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.*

In another embodiment, the promoter activating nucleic acid sequence of any of the embodiments described above is used to increase the expression level of the nucleic acid molecule of interest at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the inserted or introduced promoter activating nucleic acid sequence.

The use according to any of the embodiments described above is preferably for increasing the expression of a crop trait gene, which is preferably selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content. Specific preferred examples are ZmZEP1 (SEQ ID NO: 31), ZmRCA-beta (SEQ ID NO: 32), BvEPSPS (SEQ ID NO: 33), and BvFT2 (SEQ ID NO: 34).

### Example 1: Identification and testing of promoter activating sequences

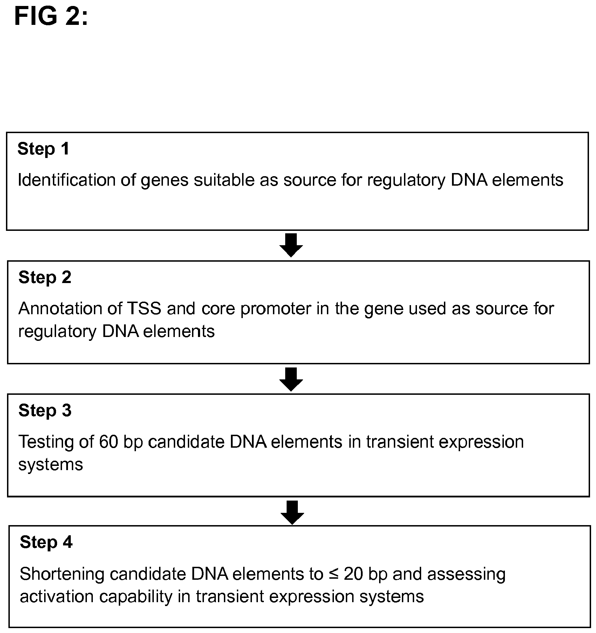

Figure 2 shows a schematic overview of the strategy for identifying and testing promoter activating DNA sequences. The process is divided into four steps:

### Step 1: Identification of genes suitable as source for regulatory DNA elements

Genes with high level of expression in most tissues und under most conditions have to be identified. This can be done by analyzing RNAseq or microarray expression data. In case of corn (Zea *mays*), such data can be found for example in Stelpflug et al., 2016 (Plant Genome. 2016 Mar;9(1). doi: 10.3835/plantgenome2015.04.0025). Exemplary, a number of genes with high level of expression has been identified in corn (Table 6a) and sugar beet (Table 6b).

**Table 6a: Corn genes suitable as source for promoter activating nucleic acid sequences**

| **gene identifier** | **Description** |
|---|---|
| GRMZM2G154397 | sam2 - S-adenosyl methionine decarboxylase2 |
| GRMZM2G091155 | orthologue to Sorghum GRF7 (GENERAL REGULATORY FACTOR 7) |
| GRMZM2G144030 | tif5A - eukaryotic translation initiation factor 5A |
| GRMZM2G102499 | grf1 - general regulatory factor1 |
| GRMZM2G116034 | eif4 - eukaryotic initiation factor4 |
| GRMZM2G108474 | PTHR11991 - Translationally controlled tumor protein related |
| GRMZM2G105996 | ADP-ribosylation factor 1 |
| GRMZM2G067985 | ACTIN // SUBFAMILY NOT NAMED |
| GRMZM2G419891 | ubi2 - ubiquitin2 |
| GRMZM2G409726 | ubi1 - ubiquitin1 |
| GRMZM2G113696 | eif5a - elongation initiation factor5A |
| GRMZM2G046804 | gpc1 - glyceraldehyde-3-phosphate dehydrogenase 1 |
| GRMZM2G180625 | gpc2 - glyceraldehyde-3-phosphate dehydrogenase2 |
| GRMZM2G134980 | rz474a(dnaj) putative chaperone |

**Table 6b: Sugar beet genes suitable as source for promoter activating nucleic acid sequences**

| **Gene ID (RefBeet 1.2)** | **Description** |
|---|---|
| g14273 | weak similarity to Extensin |
| g20480 | similarity to Glycine-rich RNA-binding protein 2 |
| g22883 | similarity to Dehydrin |
| g1084 | Adenosylhomocysteinase |
| g2331 | Calreticulin Precursor |
| g4021 | Ubiquitin |
| g3886 | Hsp90 |
| g6309 | GAPDH |
| g7096 | Actin |
| g16645 | Peroxidase |
| g10369 | Tubulin |

### Step 2: Annotation of transcription start site (TSS) and core promoter in the genes used as source for promoter activating nucleic acid sequences

The TSS and the core promoter sequences of these genes needed to be identified. Identification of TSS can be done by techniques like 5-prime tag sequencing, which are available as a service from sequencing providers like eurofins. Such a data set was generated for corn, genotype B73. Core promoter sequences can then be selected as the sequence approximately -50 to +20, preferably approximately -45 to +15 relative to the TSS (see Table 1 above). Depending on the broadness of the transcription start, the selected sequence can vary.

### Step 3: Testing of 60 bp candidate DNA elements in transient expression systems

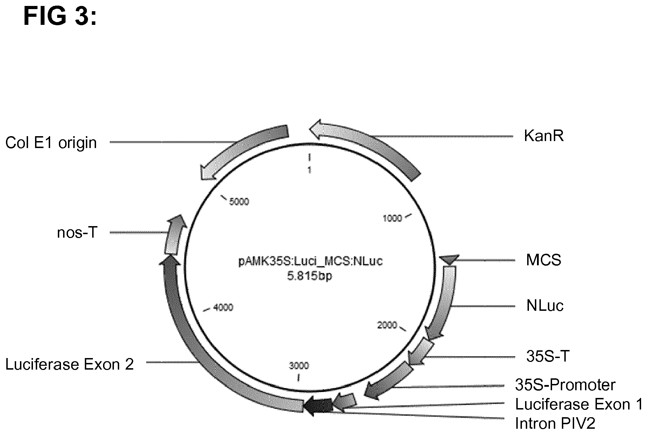

The target promoter to be activated is cloned into a reporter construct in front of a suitable reporter gene, e.g. NLuc (Masser, A. E., Kandasamy, G., Kaimal, J. M., and Andréasson, C. (2016) Luciferase NanoLuc as a reporter for gene expression and protein levels in Saccharomyces cerevisiae. Yeast, 33: 191-200. doi: 10.1002/yea.3155.) (Figure 3). A second reporter gene (Luc) under control of the 35S-promoter is used for normalization.

The 60 bp DNA elements are then inserted into the target promoter, at a position upstream or downstream of the target genes TSS which can be determined by the strategy described in step 2.

Different positions for insertion of the activating DNA element were tested. The results of these experiments allow defining the following rules for selecting insertion sites:
- The insertion site is not arbitrary. Activation depends on choosing the right insertion site.
- Selecting an insertion site too far upstream of the target gene TSS (more than 500 bp) results in loss of activation.
- Selecting an insertion site too close to the target genes start codon (~70-50 bp) results in loss of activation.
- uORFs downstream of the insertion result in loss of activation.

Transient testing by e.g. particle bombardment of leaf tissue, particle bombardment of callus, particle bombardment of root tissue, transfection of protoplasts, *Agrobacteria* mediated transient transformation allows to measure the level of activation caused by the addition of the 60 bp DNA elements. Approx. 93 % of the added sequences led to increased expression (Figure 4).

In most cases, the best performing 60 bp core promoter sequences contain a TATA box. However, our analysis revealed that there is no tight correlation between strength of the TATA box motif and the activating properties of the candidates (Table 7).

**Table 7: TATA-box motif analyses of the analyzed 60 bp candidates derived from Zea mays promoters. A relative score ≤ 0.8 indicates that no TATA box is present.**

| **Element** | **Motif ID** | **Relative score** | **start** | **end** | **motif sequence** |
|---|---|---|---|---|---|
| E53 | TATA-Box | 0,91 | 24 | 38 | CTATAAAGACAAGCC |
| E55 | TATA-Box | 0,87 | 3 | 17 | CTATAAAATATCCCC |
| E56 | TATA-Box | 0,96 | 12 | 26 | GTATAAAAAGCGGAA |
| E62 | TATA-Box | 0,81 | 5 | 19 | TCTTAAAAGCCGCCT |
| E63 | TATA-Box | 0,91 | 4 | 18 | CCATAAATGCGCCGC |
| E66 | TATA-Box | 0,87 | 10 | 24 | TCATAAATAGCCAGC |
| E67 | TATA-Box | 0,82 | 10 | 24 | TAATAAATAGACACC |
| E68 | TATA-Box | 0,91 | 12 | 26 | GTATAAATATGCTGA |
| E69 | TATA-Box | 0,90 | 6 | 20 | CTTTAAAAGGACACG |
| E70 | TATA-Box | 0,89 | 6 | 20 | CTTTAAAAACGCACA |

Nevertheless, the experiments showed that in most cases the ≤ 20 bp DNA elements containing the TATA box motif is the element with the strongest activation properties. Therefore, it would also be possible to directly select and test 20 bp candidate DNA elements containing the TATA box motif instead of starting with 60 bp sequences. However, not all activating 60 bp DNA elements contain a TATA box, therefore TATA-less 20 bp elements are also possible.

### Step 4: Shortening candidate DNA elements to ≤ 20 bp and assessing activation capability in transient expression

A 20 bp candidate sequence can be identified by deletion analysis. Deletion constructs were made for several of the best performing 60 bp sequences, ideally by repeatedly deleting 10 bases from the 5' and the 3' end. These shortened sequences are then tested by the same strategy as described for the 60 bp sequences in step 3.

Shortening of sequences can cause the loss of activating potential. By this stepwise approach ≤ 20 bp sequences which retain a high activating potential were identified. Using this approach, the novel 20 bp activating elements E53b, E55a and E56a were identified and it was demonstrated that it is possible to further shorten these sequences while retaining activating potential (see Table 2 above).

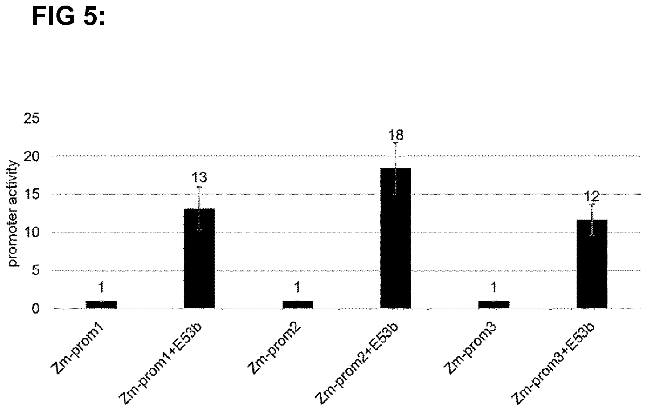

The elements identified by this approach can activate different target genes in a comparable manner. As an example, the element E53b was inserted into the promoters of 3 different corn trait genes. Activation of these trait genes was measured in a transient assay, bombardment of corn leaves. The Zm promoter1 was activated 13-fold, the Zm promoter2 was activated 18-fold and the Zm promoter3 was activated 12-fold by E53b (Figure 5).

The activation properties of identified elements can be further optimized by selecting the optimal 20 bp frame (see E53e in Table 3) or by combining different parts of the initial 60 bp sequence (see E53f in Table 3 combining 10 bp element containing the TATA box motif with a 10 bp element of the 5' end). As an example, optimized versions of element E53b are displayed in Table 3.

### Example 2: Stable Agrobacteria mediated transformation of Zea mays to characterize expression activating DNA elements in the genomic context by using luciferase reporters

To analyze the expression activating effect of small DNA elements in the context of genomic DNA and chromatin structure, stable transformation of corn by Agrobacteria is performed. The binary vectors used and the methods for analysis of transgenic corn plants are described in the following.

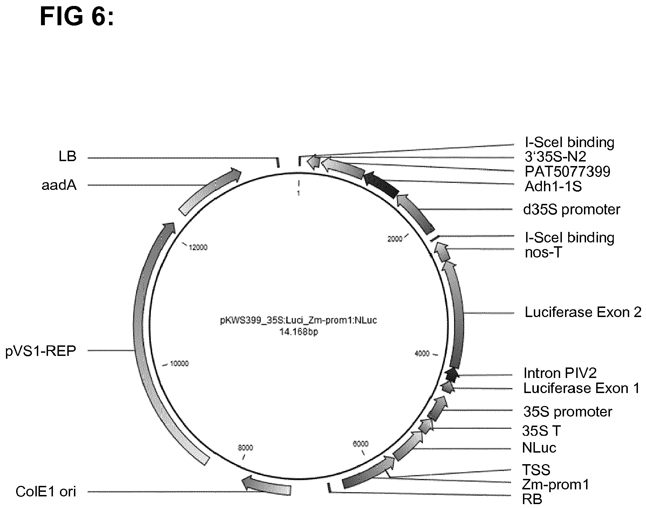

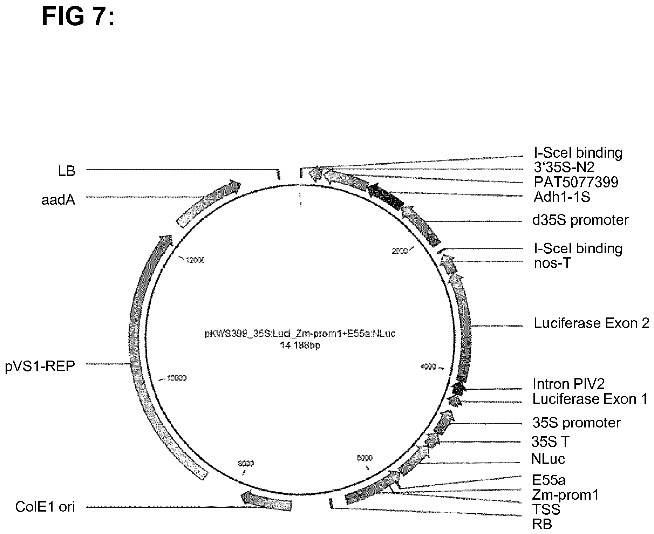

The promoter Zm-prom1 or the modified promoter Zm-prom1+E55a drives expression of the reporter NLuc (NanoLuciferase) which allows for measurement of Zm-prom1 activity in corn transformants by luciferase assay. In the modified promoter Zm-prom1+E55a the activating DNA element E55a is integrated 88 bp downstream of the original TSS (transcription start site) and 122 bp upstream of the NLuc start codon. Normalization of the NLuc signal is possible due to the presence of a second reporter Luciferase which is expressed under control of the 35S-promoter. Such a system enables the careful evaluation of Zm-prom1 or Zm-prom1+E55a activity despite of effects based on different insertion sites in the corn genome. The PAT-gene functions as selection marker. Transgenic corn plants transformed with the constructs pKWS399_35S:Luci_Zm-prom1 :NLuc (Figure 6) and pKWS399_35S:Luci_Zm-prom1+E55a:NLuc (Figure 7) are analyzed for NLuc and Luciferase activity as well as for transcript levels of NLuc and Luciferase.

### Example 3: Stable Agrobacteria mediated transformation of Zea mays to characterize expression activating DNA elements in the genomic context bv assessing corn gene expression

To analyze the expression activating effect of small DNA elements in the context of genomic DNA and chromatin structure, stable transformation of corn by Agrobacteria is performed. The binary vectors used and the methods for analysis of transgenic corn plants are described in the following.

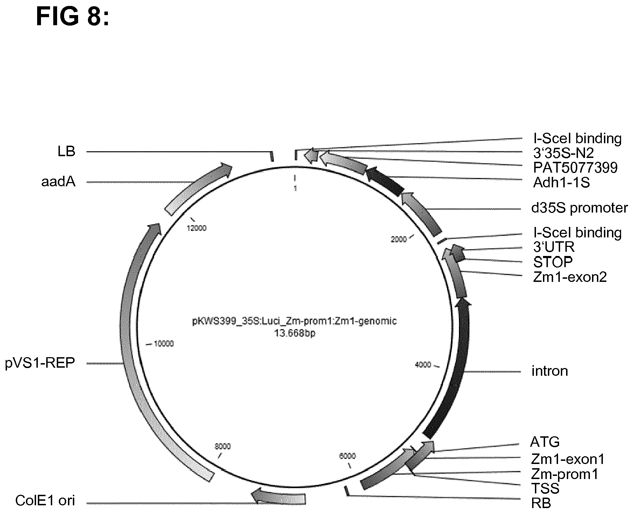

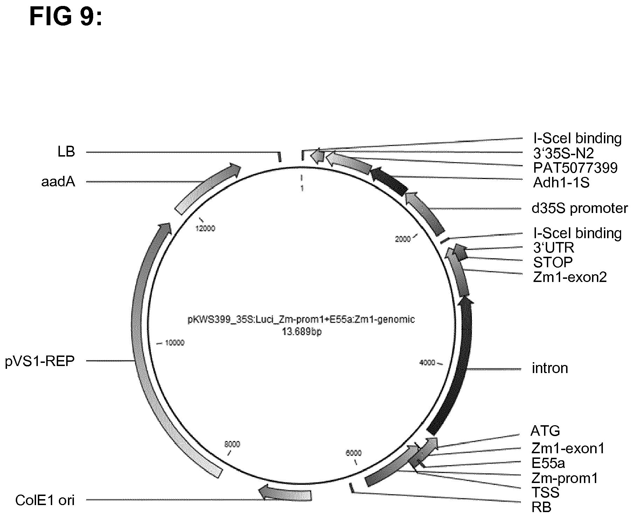

The promoter Zm-prom1 or the modified promoter Zm-prom1+E55a drives expression of its own endogenous corn gene Zm1. The whole genomic locus of Zm1 was cloned. In the modified promoter Zm-prom1+E55a the activating DNA element E55a is integrated 88 bp downstream of the original TSS (transcription start site) and 111 bp upstream of the Zm1 start codon. The PAT-gene functions as selection marker. Transgenic corn plants transformed with the constructs pKWS399_35S:Luci_Zm-prom1:Zm1-genomic (Figure 8) and pKWS399_35S:Luci_Zm-prom1+E55a:Zm1-genomic (Figure 9) are analyzed for Zm1 transcript and protein levels.

### Example 4: Targeted insertion of small activating DNA elements (one circular vector)

For targeted insertion of small activating DNA elements by homologous recombination (HR) a construct carrying the element (e.g E55a) to be inserted, flanked by suitable homology regions of the respective promoter into which the element should be inserted (e.g Zm-prom1) may be used. In principle, any target region, promoter of interest or even a nucleic acid to be altered of interest, in the genome of a cell of interest may be used. Here the exemplary target promoter is the promoter Zm-prom1. Instead of element E55a, another small activating DNA element may be used.

In addition, the vector contains a CRISPR nuclease, including *inter alia* a Cas or Cpf, CasX or CasY, encoding sequence as effector nuclease and a corresponding sgRNA or crRNA aligning with the specific region in the target promoter Zm-prom1 where the insertion is supposed to occur. Gene editing of WT corn plants could either be performed by using stable Agrobacteria mediated transformation followed by later segregating away the gene editing tools or in a transient way.

To check if a HR-based repair has occurred, plants can be easily analyzed by PCR and amplicon sequencing based on the available sequence information. To verify the activating of effect of the small DNA element expression studies on transcript and/or protein level are done.

### Example 5: Targeted insertion of small activating DNA elements (two circular vectors)

For targeted insertion of small activating DNA elements by homologous recombination a construct carrying the element (e.g E55a) to be inserted, flanked by suitable homology regions of the respective promoter into which the element should be inserted (e.g Zm-prom1) may be used. In principle, any target region, promoter of interest or even a nucleic acid to be altered of interest, in the genome of a cell of interest may be used. Here the exemplary target promoter is the promoter Zm-prom1. Instead of element E55a, another small activating DNA element may be used.

In addition, a second vector encoding a Cas or Cpf effector, or any other CRISPR nuclease, as site-specific nuclease and a sgRNA/crRNA aligning with the specific region in the target promoter Zm-prom1 where the insertion is supposed to occur may be used. Gene editing of WT corn plants could either be performed by using stable Agrobacteria mediated transformation followed by later segregating away the gene editing tools or in a transient way.

To check if a HR-based repair has occurred, plants can be easily analyzed by PCR and amplicon sequencing based on the available sequence information. To verify the activating of effect of the small DNA element expression studies on transcript and/or protein level are done.

### Example 6: Conversion of original TATA-box into TATA-box from activating DNA element

The original TATA-box of a promoter of interest (e.g Zm-prom1) is exchanged for the specific TATA-box being part of a small activating DNA element (e.g E59). The exchange is positioned 23 bp upstream of the TSS (given with bases TGA in this case).
original Zm-prom1 TATA-box:
   TTATTATTANNNNNNNNNNNNNNNNNNNNNNNTGA (SEQ ID NO: 35)
original Zm-prom1 TATA-box converted to E59 (Zm-prom1v3):
   GTATAAAAGNNNNNNNNNNNNNNNNNNNNNNNTGA (SEQ ID NO: 36)

The effect is measured in a transient assay system based on corn leaf bombardment with respective promoter-reporter constructs followed by luciferase measurement.

The modified promoter Zm-prom1v3 is 9,75-fold activated compared to the unmodified Zm-prom1. For comparison the result from insertion of E59 further downstream of the original TSS (Zm-prom1+E59) is displayed in addition (see Figure 10A).

### Example 7: Base editing to generate sequence of an activating DNA element

Base editors coupled to a catalytically impaired Cas or Cpf effector, or any other CRISPR nuclease can mediate targeted transitions of C-G to T-A and of A-T to G-C by either using a cytosine deaminase or an adenine deaminase evolved to process DNA (Gaudelli et al., Nature, 551, 464-471, November 2017).

These tools are used to convert the sequence of the promoter of interest (e.g Zm-prom1) at a position suitable for activation into the sequence of a small activating DNA element. This could exemplary either be conversion of the original TATA-box of the promoter of interest into the specific TATA-box being part of a small activating DNA element or this can be other base pairs at positions surrounding the core promoter which is suitable for introduction of an activating DNA element. The sequence to be restored can be a small activating DNA element or only part of it.

### Example 8: Conversion of original TATA-box into activating DNA elements

The original TATA-box of a promoter of interest (e.g ZmZEP1) is converted into small activating DNA element (e.g E59, E53f, E55a) by using site specific mutagenesis (base editing). The exchange is positioned -33 bp upstream of the TSS (given with bases CAA in this case).
original ZmZEP1 TATA-box:
   AAGATAAAATCCTGGTCCAGCAAGATCCGTTCTTCCAA (SEQ ID NO: 37)
original ZmZEP1 TATA-box converted to activating DNA elements E59 (ZmZEP1 v1):
   AAGTATAAAAGTCCTGGTCCAGCAAGATCCGTTCTTCCAA (SEQ ID NO:38)
original ZmZEP1 TATA-box converted to activating DNA elements E53f (ZmZEP1v2):
   AAGCTATAAAGAGCATCCCTTCAAGATCCGTTCTTCCAA (SEQ ID NO: 39)
original ZmZEP1 TATA-box converted to activating DNA elements E55a (ZmZEP1v3):
   AAGCTATAAAATATCCCCACGCAAGATCCGTTCTTCCAA (SEQ ID NO: 40)

The effect is measured in a transient assay system based on corn leaf bombardment with respective promoter-reporter constructs followed by luciferase measurement (see Figure 10C).

The modified promoter ZmZEP1v1 is activated 5,9-fold, the modified promoter ZmZEP1v2 is activated 15,6-fold and the modified promoter ZmZEP1v3 is activated 10,5-fold compared to the unmodified ZmZEP1 promoter. For comparison the result for insertion of E53b further downstream of the original TSS (ZmZEP1+E53b) is displayed in addition.

The element E59 consists of a TATA-box only which was deduced from the matrix model deposited in the JASPAR database [http://jaspar.genereg.net/]. The TATA-box version E59a represents the perfect consensus sequences for a monocot TATA-box (see also Figure 11C) and the versions E59b-d are slightly modified by taking the matrix model into account [Shahmuradov IA, Gammerman AJ, Hancock JM, Bramley PM, Solovyev VV (2003) PlantProm: a database of plant promoter sequences. Nucleic acids research 31: 114-117).

In Figure 10B the comparison of the activating capability of elements E59 and E59a-d (see Table 4) when inserted into a corn target promoter clearly show that element E59a confers the highest activation. This is in line with element E59a representing the the perfect consensus sequences for a monocot TATA-box.

## Claims

1. A promoter activating nucleic acid sequence configured for targeted site-specific insertion into a recipient promoter controlling the expression of a nucleic acid molecule of interest in a cell or an organism, wherein the promoter activating nucleic acid sequence causes an increased expression of the nucleic acid molecule of interest upon site-specific insertion, preferably wherein the nucleic acid molecule of interest is heterologous or native to the recipient promoter and/or is an endogenous or exogenous nucleic acid molecule to the cell or organism.

2. The promoter activating nucleic acid sequence according to claim 1, wherein the promoter activating nucleic acid sequence has a length between 6 and 70 nucleotides, preferably between 7 and 60 nucleotides, more preferably between 8 and 40 nucleotides and most preferably between 9 and 20 nucleotides.

3. The promoter activating nucleic acid sequence according to claim 1 or 2, comprising
i. one or more contiguous stretch(es) of nucleotides isolated from a donor promoter, wherein the donor promoter is a promoter of a gene having a high expression level, and/or
ii. one or more TATA box motif(s) of a donor promoter or one or more TATA box motif(s) having a relative score of greater than 0.8 when matching or aligning the one or more TATA box motif(s) to TATA box consensus, and/or
iii. comprises one or more pyrimidine patch (Y patch) promoter element(s) of a donor promoter.

4. The promoter activating nucleic acid sequence according to any of the previous claims, wherein the promoter activating nucleic acid sequence has a sequence identity of at least 75% to one of the sequences of SEQ ID NOs: 1 to 30, GTATAAAAG (E59), CTATAAATA (E59a), CTATATATA (E59b), CTATAAAAA (E59c) and CTATATAAA (E59d) over the whole length of the promoter activating nucleic acid sequence.

5. The promoter activating nucleic acid sequence according to any of the previous claims, wherein the cell or organism is a plant cell or plant, and/or wherein the recipient promoter and/or the donor promoter is/are a plant promoter, and/or wherein the recipient promoter and the donor promoter are different and/or originate from the same species or from different species.

6. The promoter activating nucleic acid sequence according to any of the previous claims, wherein upon site-specific insertion into the recipient promoter the expression level of the nucleic acid molecule of interest is increased at least 2-fold, at least 3-fold, at least 4-fold or at least 5-fold, preferably at least 6-fold, at least 7-fold, at least 8-fold, at least 9-fold or at least 10-fold, more preferably at least 12-fold, at least 14-fold, at least 16-fold, at least 18-fold or at least 20-fold, even more preferably at least 25-fold, at least 30-fold, at least 35-fold or at least 40-fold and most preferably more than 40-fold, compared to the expression level of the nucleic acid molecule of interest under the control of the recipient promoter without the insertion.

7. A chimeric promoter comprising a recipient promoter or the core promoter thereof and at least one promoter activating nucleic acid as defined in any one of the claims 1 to 6 at a position upstream or downstream of the transcription start site of the recipient promoter.

8. A delivery system comprising the promoter activating nucleic acid sequence according to any of claims 1 to 6 and/or the chimeric promoter of claim 7, and/or means for site-specific insertion or introduction of the promoter activating nucleic acid sequence as defined in any of claims 1 to 6 into a recipient promoter.

9. A nucleic acid construct or an expression cassette comprising the promoter activating nucleic acid sequence according to any of claims 1 to 6 and/or the chimeric promoter of claim 7.

10. A vector comprising the promoter activating nucleic acid sequence according to any of claims 1 to 6, the chimeric promoter of claim 7 or the nucleic acid construct or the expression cassette according to claim 9, or means for site-specific introduction of the promoter activating nucleic acid sequence as defined in any of claims 1 to 6, the chimeric promoter of claim 7 or the nucleic acid construct or the expression cassette according to claim 9 into a recipient promoter.

11. A cell or organism or a progeny thereof or a part of the organism or progeny thereof,
a) in which a promoter activating nucleic acid as defined in any one of claims 1 to 6 is inserted or introduced by addition and/or deletion and/or substitution of one or more nucleotides into a recipient promoter controlling the expression of a nucleic acid molecule of interest in the cell or the organism, preferably inserted or introduced at a position upstream or downstream of the transcription start site of the recipient promoter, more preferably at a position
i. 500 nucleotides or less, preferably 150 nucleotides or less nucleotides upstream of the transcription start site of the nucleic acid molecule of interest, and/or
ii. 50 or more nucleotides upstream of the start codon of the nucleic acid molecule of interest; and/or
iii. where there is no upstream open reading frame (uORF) downstream of the insertion or introduction site, or
b) comprising the chimeric promoter according to claim 7, the delivery system according to claim 8, the nucleic acid construct or an expression cassette according to claim 9 or the vector according to claim 10.

12. A method for identifying a promoter activating nucleic acid sequence or a chimeric promoter, comprising:
i) identifying a gene in a cell or an organism having a high expression level,
ii) isolating one or more contiguous stretch(es) from the promoter of the gene identified in step i) wherein the one or more contiguous stretch(es) originate(s) a) from the core promoter of the said donor promoter or b) from a sequence from position -50 to position +20 relative to the transcription start site of said donor promoter,
iii) inserting or introducing by addition and/or deletion and/or substitution of one or more nucleotides the one or more contiguous stretch(es) into a recipient promoter controlling the expression of a nucleic acid molecule of interest at a position upstream or downstream of the transcription start site of the recipient promoter,
iv) determining in a cell or organism or *in vitro* the expression level of the nucleic acid molecule of interest under the control of the recipient promoter comprising the insertion or introduction of step iii) relative to the expression level of the same or another nucleic acid molecule of interest under the control of the recipient promoter without the insertion or introdcution of step iii) or to another reference promoter in a given environment and/or under given genomic and/or environmental conditions, wherein the nucleic acid molecule of interest is heterologous or native to the recipient promoter and/or is endogenous or exogenous to the cell or organism to the cell or organism, and
v) identifying and thus providing the promoter activating nucleic acid sequence as defined in any one of claims 1 to 6 or the chimeric promoter according to claim 7 when increased expression of the nucleic acid molecule of interest in step iv) is observed,
vi) optionally, shortening the promoter activating nucleic acid sequence identified in step v) stepwise and repeating steps iv) and v) at least one time and/or modifying one or more TATA box motif(s) present in the promoter activating nucleic acid sequence identified in step v) or in the recipient promoter by addition and/or substitution and/or deletion of one or more nucleotides for converting the one or more TATA box motif(s) into one or more TATA box motif(s) having increased or higher relative score(s) when matching or aligning the one or more TATA box motif(s) to the TATA box consensus, and repeating steps iv) and v) at least one time.

13. Method for increasing the expression level of a nucleic acid molecule of interest in a cell, preferably a plant cell comprising:
ia) introducing into the cell the promoter activating nucleic acid sequence according to any one of claims 1 to 6, the chimeric promoter according to claim 7, the delivery system according to claim 8, or the nucleic acid construct or expression cassette according to claim 9, or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any of claims 1 to 6 into a recipient promoter controlling the expression of the nucleic acid molecule of interest in the cell at a position upstream or downstream of the transcription start site of the recipient promoter controlling the expression of the nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of the nucleic acid molecule of interest by addition and/or deletion and/or substitution so that a promoter activating nucleic acid sequence as defined in any of claims 1 to 6 is formed, and
iiic) optionally, modifying one or more TATA box motif(s) present in the promoter activating nucleic acid sequence inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus.

14. Method for producing a cell or organism having increased expression level of a nucleic acid molecule of interest, comprising:
ia) introducing into the cell the promoter activating nucleic acid sequence according to any one of claims 1 to 6, the chimeric promotor according to claim 7, the delivery system according to claim 8, or the nucleic acid construct or an expression cassette according to claim 9, or
ib) introducing into the cell means for site-specific modification of the nucleic acid sequence of a recipient promoter controlling the expression of the nucleic acid molecule of interest, and
ii) optionally, introducing into the cell a site-specific nuclease or an active fragment thereof, or providing the sequence encoding the same, the site-specific nuclease inducing a double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and
iiia) inserting the promoter activating nucleic acid sequence as defined in any one of claims 1 to 6 or the chimeric promotor as defined in claim 7 into a recipient promoter controlling the expression of the nucleic acid molecule of interest in the cell at a position upstream or downstream of the transcription start site of the recipient promoter controlling the expression of a nucleic acid molecule of interest, or
iiib) modifying the sequence of a recipient promoter controlling the expression of the nucleic acid molecule of interest in the cell at a position upstream or downstream of the transcription start site of the recipient promoter controlling the expression of a nucleic acid molecule of interest by addition and/or deletion and/or substitution so that a promoter activating nucleic acid sequence as defined in any of claims 1 to 6 is formed, and
iiic) optionally, modifying one or more TATA box motif(s) present in the promoter activating nucleic acid sequence or the chimeric promoter inserted or introduced in step iiia) or iiib) or present in the recipient promoter by addition and/or substitution and/or deletion of one or more nucleotides for converting the one or more TATA box motif(s) into one or more TATA box motif(s) having increased or higher relative score(s) when matching or aligning the one or more modified TATA box motif(s) to the TATA box consensus, and
iv) obtaining a cell or organism having increased expression level of a nucleic acid molecule of interest upon insertion of the promoter activating nucleic acid sequence as defined in any of claims 1 to 6 or upon modification to form the promoter activating nucleic acid sequence as defined in any of claims 1 to 6.

15. A cell or organism or a progeny thereof, preferably a plant cell or plant or progeny thereof, obtainable by a method of claim 14.
